# EUROPEAN PATENT APPLICATION

(11) **EP 4 467 153 A1**
(43) Date of publication of application: **27.11.2024**
(21) Application number: 23386040.2
(22) Date of filing: 24.05.2023
(51) Int. Cl.: A61K 38/12, A61P 25/16, C07K 4/12, C07K 7/64

(54) **CONSTRAINED PEPTIDE INHIBITORS OF LRRK2-FADD INTERACTIONS**

(71) Applicant: University of Georgia Research Foundation, Inc., Athens, Georgia 30602 (US); Rideout, Hardy, 115 26 Athens (GR)
(72) Inventor: RIDEOUT, Hardy, 115 26 Athens (GR); KENNEDY, Eileen J., Athens, Georgia 30605 (US); HELTON, Leah G., Chattanooga, Tennessee 37412 (US)
(74) Representative: Dehns

(57) **Abstract**

Synthetic polypeptides are described comprising an amino acid sequence having an α-helical shape that mimics the death domain of FADD, wherein the polypeptides comprise at least one pair of non-natural amino acids inserted into the amino acid sequence, wherein the pair of non-natural amino acids are cross-linked to stabilize the α-helical shape. Methods of treating medical disorders using the synthetic polypeptides are also described.

## Description

### TECHNICAL FIELD

This disclosure relates to peptides useful in treating medical disorders and, more particularly, to constrained peptides capable of inhibiting or modulating the interaction of LRRK2 and FADD.

### BACKGROUND

Neuronal death induced by mutant forms of leucine-rich repeat kinase 2 (LRRK2), as demonstrated in viral *in vivo* or cellular over-expression models, is the result of a complex series of signaling events requiring intact kinase activity (for example, see Greggio E, Jain S, Kingsbury A, Bandopadhyay R, Lewis P, Kaganovich A, van der Brug MP, Beilina A, Blackinton J, Thomas KJ, Ahmad R, Miller DW, Kesavapany S, Singleton A, Lees A, Harvey RJ, Harvey K, Cookson MR (2006) Kinase activity is required for the toxic effects of mutant LRRK2/dardarin. Neurobiol Dis 23, 329-341) as well as specific components of various cell death pathways (see Antoniou N, Vlachakis D, Memou A, Leandrou E, Valkimadi PE, Melachroinou K, Re DB, Przedborski S, Dauer WT, Stefanis L, Rideout HJ (2018) A motif within the armadillo repeat of Parkinson's-linked LRRK2 interacts with FADD to hijack the extrinsic death pathway. Sci Rep 8, 3455; and Ho CC, Rideout HJ, Ribe E, Troy CM, Dauer WT (2009) The Parkinson disease protein leucine-rich repeat kinase 2 transduces death signals via Fas-associated protein with death domain and caspase-8 in a cellular model of neurodegeneration. J Neurosci 29, 1011-1016). The baseline interaction between wild-type (WT) LRRK2 and the death adaptor protein FADD is strengthened by most pathogenic mutant forms of LRRK2, and this interaction serves to recruit and activate caspase-8. The increased interaction between mutant LRRK2 and FADD was downstream of LRRK2 kinase activity. Genetic ablation of kinase activity restored the interaction with FADD to WT LRRK2 levels. In addition to activating these components of the extrinsic death pathway, FADD/caspase-8 signaling in neurons activated downstream mitochondrial death cascades involving translocation of Bid and Bax to the mitochondria.

The binding domain of LRRK2 that mediates the interaction with the FADD death-domain to the armadillo region within the large N-terminal domain of LRRK2 has been mapped previously. Homology modeling of this region of LRRK2, coupled with molecular docking techniques, further refined the domain mapping to identify candidate residues that mediate this interaction. Targeting the LRRK2-FADD interaction using genetic or dominant negative approaches successfully reduced the PPI and was neuroprotective in primary cultured cortical neurons. Deletion of the FADD-binding region within the ARM domain of LRRK2 reduced the recruitment of WT LRRK2 to over-expressed FADD death-effector filaments and prevented the induction of neuronal death by mutant LRRK2. Similarly, co-expression of mutant LRRK2 and a dominant negative fragment of the FADD death-domain prevented the binding of LRRK2 with full-length FADD and was also neuroprotective.

Mutations in the gene encoding leucine-rich repeat kinase 2 (*LRRK2*) are the most common cause of familial Parkinson's disease (PD). The reduced penetrance of mutations in the *LRRK2* gene has also led to variants appearing in seemingly sporadic forms of the disease. Kinase inhibition effectively blocks neuronal death, and small molecule Class I inhibitors are proceeding through clinical trials in multiple PD cohorts. The toxic interaction between mutant LRRK2 and FADD lies downstream of its kinase activity and is required to induce neuronal death.

There is a clear need for inhibitors or modulators of the interaction between FADD and LRRK2.

### SUMMARY

The present disclosure provides constrained peptides that mimic the death domain of FADD that interacts with the armadillo region of LRRK2. The constrained peptides are useful in treating medical disorders such as neurological diseases, disorders, or conditions, for example, Parkinson's disease.
In one aspect, a synthetic polypeptide is provided comprising an amino acid sequence having an α-helical shape that mimics the death domain of FADD, wherein the polypeptide comprises at least one pair of non-natural amino acids inserted into the amino acid sequence, wherein the pair of non-natural amino acids are cross-linked to stabilize the α-helical shape.

In another aspect, a pharmaceutical composition is provided comprising the synthetic polypeptide described herein in a pharmaceutically acceptable carrier.

In another aspect, a cell is provided comprising the synthetic polypeptide described herein.

In another aspect, a method is provided for treating a neurological disease, disorder, or condition in a subject in need thereof comprising administering to the subject a therapeutically effective amount of a polypeptide described herein.

In another aspect, a method is provided of treating a disease, disorder, or condition in a subject that is treated by inhibiting or decreasing protein-protein interactions between LRRK2 and FADD comprising administering to the subject a therapeutically effective amount of a polypeptide described herein.

The details of one or more embodiments of the disclosure are set forth in the accompanying drawings and the description below. Other features, objects, and advantages of the disclosure will be apparent from the description and drawings and from the claims.

### DESCRIPTION OF DRAWINGS

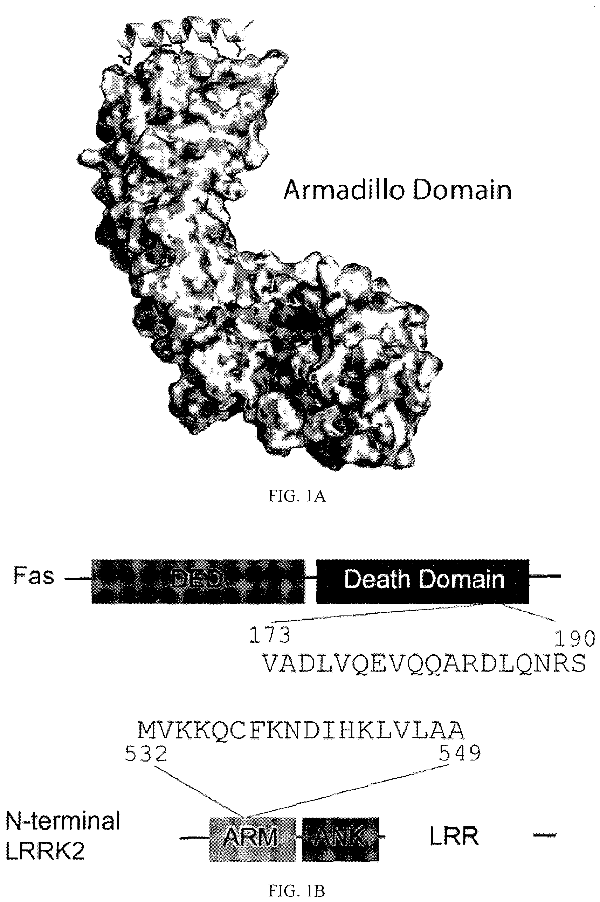

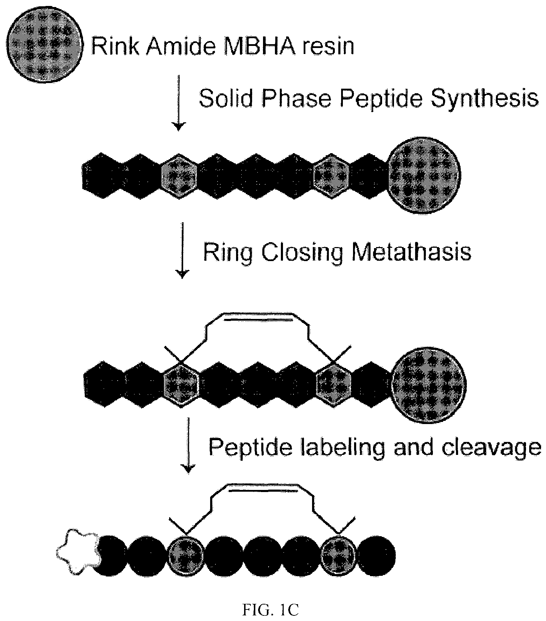

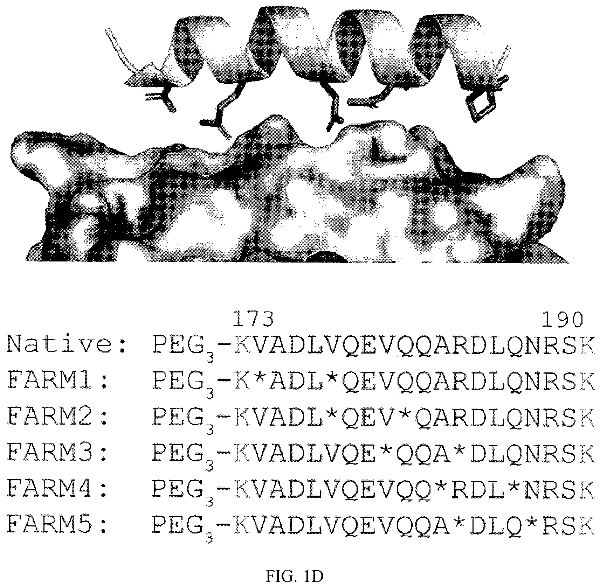

**FIGs. 1A-1D** depict the design and structure of constrained peptides targeting the LRRK2-FADD PPI. **(****FIG. 1A****)** Structural representation of the region of the LRRK2 ARM domain in which the FADD-binding motif is located, shown with the fragment of the FADD death domain that binds LRRK2. **(****FIG. 1B****)** Highlight schematic of the relevant binding domains within LRRK2 and FADD, and the corresponding amino acid sequence that mediates this PPI is highlighted. **(****FIG. 1C****)** Schematic representation of the solid phase peptide synthesis, highlighting the placement of the synthetic amino acids for the placement of the peptide staple. **(****FIG. 1D****)** Sequences of the five stapled peptides synthesized, with the position of the synthetic residues in each peptide indicated by an asterisk.

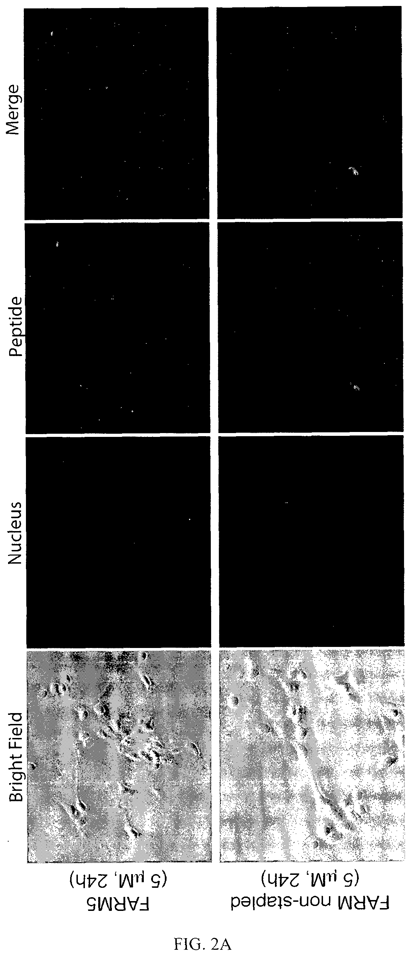

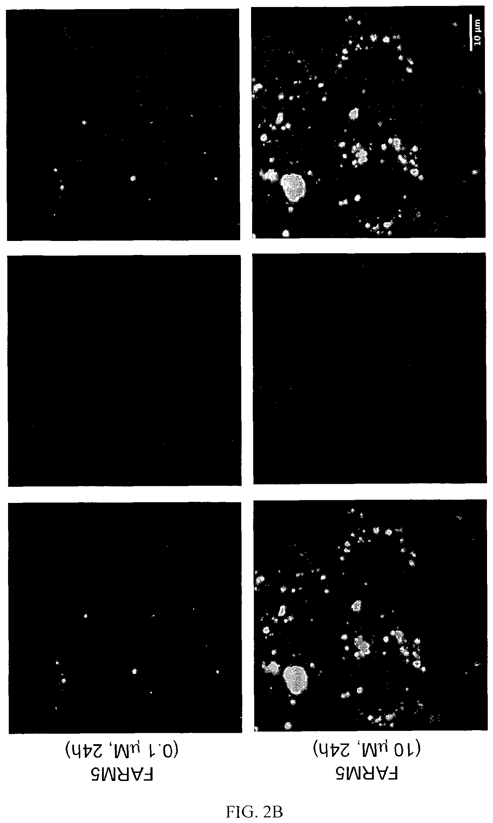

**FIGs. 2A-2B** depict the cellular uptake of stapled vs. native peptides. **(****FIG. 2A****)** HEK293T cells were incubated with 5 µM of non-stapled control or constrained FARMS for a period of 24 hr. The cells were fixed and counterstained for nuclei (DAPI) and mounted on slides for imaging. The presence of the peptide staple improved the cellular permeation of the FAM-labeled fluorescent peptide, which was distributed diffusely throughout the cell with occasional puncta visible as well. **(****FIG. 2B****)** Higher magnification confocal image of HEK293T cells treated for 24hr with 0.1 or 10 µM of FAM-labeled FARMS, followed by fixation and counter-staining with the nuclear dye DAPI. The peptides are distributed throughout the cell, with greater numbers of puncta observed in the cytoplasm.

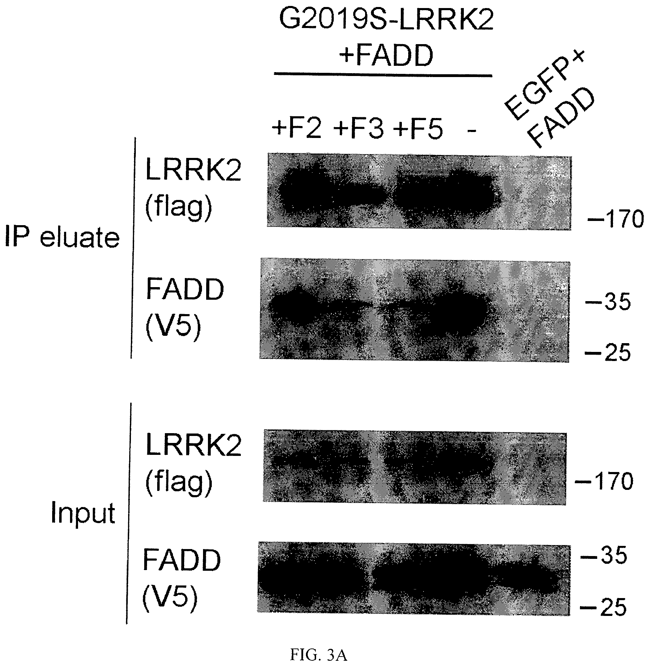

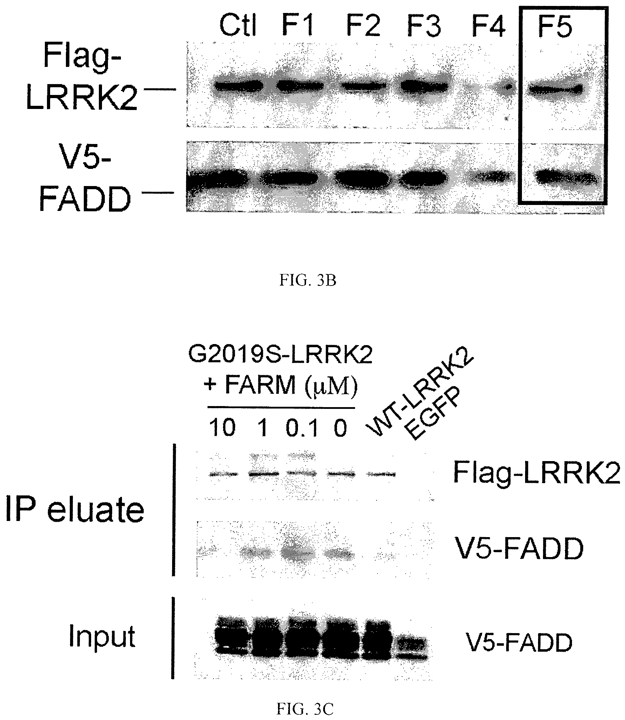

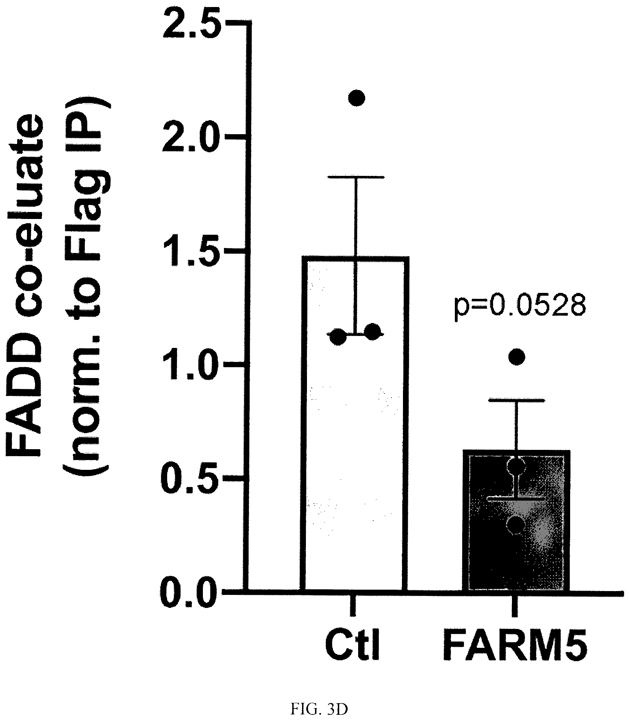

**FIGs. 3A-3D** depict the disruption of the LRRK2-FADD PPI by targeted stapled peptides. **(****FIGs. 3A** **and** **3B****)** HEK293T cells were transiently transfected with Flag G2019S-LRRK2 and V5-tagged FADD. Forty-eight hr after transfection, the cells were treated with vehicle or the indicated peptide (10µM) and incubated for a further 48hr. The cells were washed and processed for co-immunoprecipitation using Flag antibody-coated resin. The eluate was probed for precipitated LRRK2 (Flag) and co-eluting FADD (V5). Both FARM3 and FARMS reduced the interaction between LRRK2 and FADD when normalized to the amount of Flag-LRRK2 precipitated and, to a lesser extent FARM2, compared to control-treated cells. **(****FIG. 3C****)** HEK293T cells were again co-transfected with Flag-LRRK2 and V5-FADD, followed by treatment with increasing doses of FARMS for an additional 24hr. In the eluate, a dose-dependent reduction of FADD was detected with increasing concentrations of FARMS. **(****FIG. 3D****)** The band intensities from **FIGs. 3A-3C** in samples treated with 10 µM FARM5 show the relative levels of FADD co-eluting with LRRK2; p=0.528.

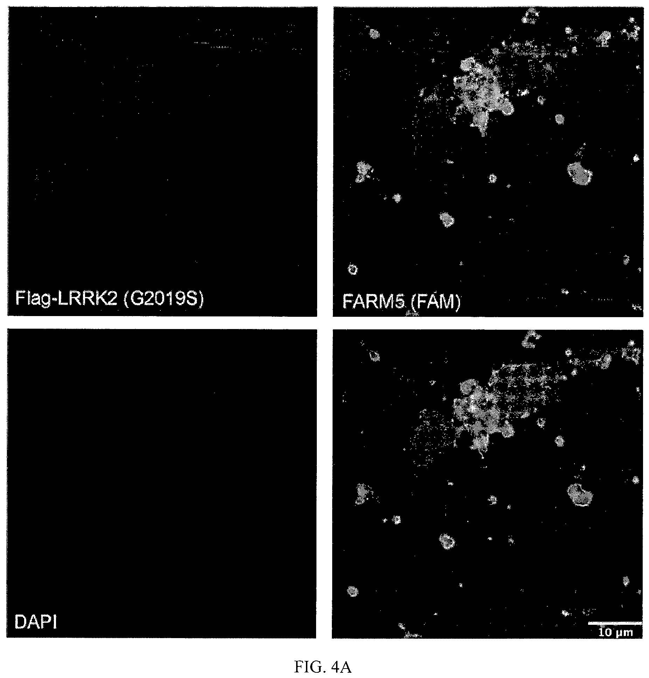

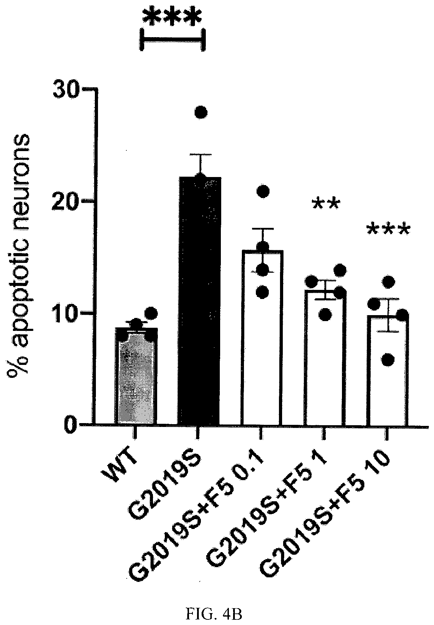

**FIGs. 4A-4B** depict that stapled peptides block apoptotic death of neurons expressing G2019S-LRRK2. **(****FIG. 4A****)** Primary embryonic cortical neurons were transiently transfected with Flag-LRRK2 (WT or G2019S) and subsequently treated with the indicated concentrations of FARMS. The peptide was replenished after 24hr, and the total treatment period was 48hr. The neurons were fixed and processed for anti-Flag immunostaining with DAPI as a nuclear counterstain. Shown is a representative confocal micrograph of neurons expressing G2019S-LRRK2 (Flag) and treated with 1 µM FARMS (FAM). **(****FIG. 4B****)** The percentage of transfected neurons with apoptotic nuclear features was determined in a blinded fashion. The plots represent the mean apoptotic neurons (+/-SEM) from 4 technical replicates of one of 3 biological replicates (independent culture preps). ** p<0.01; *** p<0.001, ANOVA with Tukey's HSD post-hoc test for pairwise comparisons.

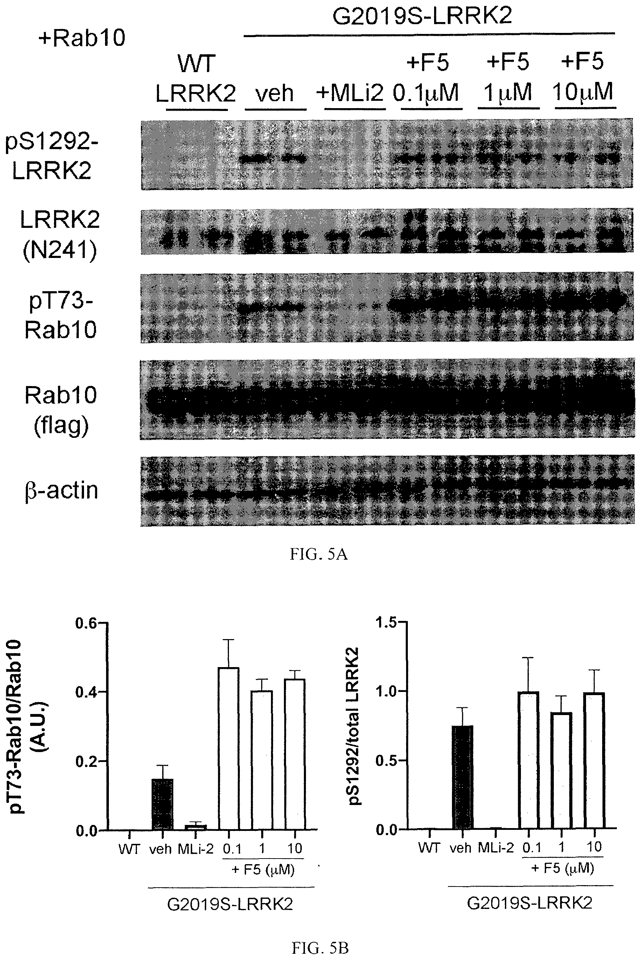

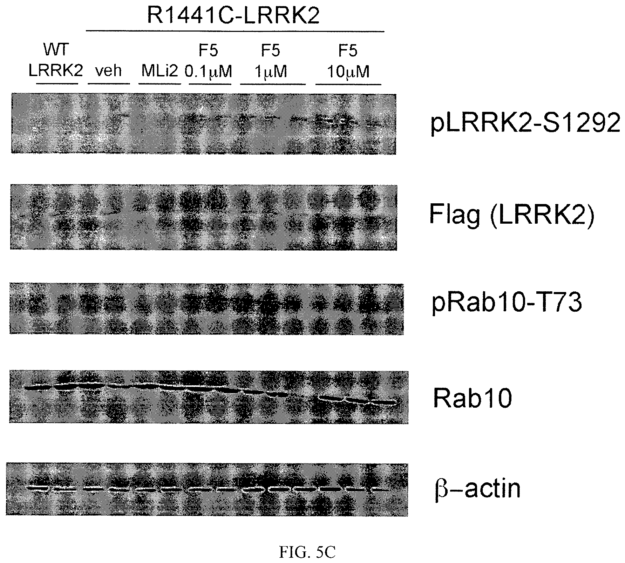

**FIGs. 5A-5C** depict that LRRK2-FADD PPI disruptors do not alter intrinsic kinase activity of LRRK2. **(****FIG. 5A****)** HEK293T cells were co-transfected with pcms-LRRK2 cDNA (WT or G2019S) and Flag-Rab10, and 48hr later treated with the indicated concentration of FARMS for 24hr, or MLi-2 (100nM, 12hr). The cell extracts were separated by SDS-PAGE, and the membranes probed for total and pS1292-LRRK2 or anti-Flag (total RablO) and pT73-Rab10 antibodies. **(****FIG. 5B****)** The band intensities in **FIG. 5A** were quantified using Image J (n=2). The signals from pS1292-LRRK2 and pT73-Rab10 were normalized to the respective total antibody signal (N241-LRRK2 and Flag, respectively). **(****FIG. 5C****)** Phosphorylation of endogenous RablO in HEK293T cells by over-expressed WT or R1441C-LRRK2 was determined by Western blot, as in **FIG. 5A****.** As observed for over-expressed RablO, auto-phosphorylation of S1292-LRRK2 or endogenous RablO by R1441C-LRRK2 was blocked by treatment with MLi-2 (100nM), but not FARMS.

Like reference symbols in the various drawings indicate like elements.

### DETAILED DESCRIPTION

The following description of the disclosure is provided as an enabling teaching of the disclosure in its best, currently known embodiments. Many modifications and other embodiments disclosed herein will come to mind to one skilled in the art to which the disclosed compositions and methods pertain, benefiting from the teachings presented in the descriptions herein and the associated drawings. Therefore, it is understood that the disclosures are not limited to the specific embodiments disclosed and that modifications and other embodiments are intended to be included within the scope of the appended claims. The skilled artisan will recognize many variants and adaptations of the aspects described herein. These variants and adaptations are intended to be included in the teachings of this disclosure and to be encompassed by the claims herein.

Although specific terms are employed herein, they are used in a generic and descriptive sense only and not for purposes of limitation.

As apparent to those of skill in the art upon reading this disclosure, each of the individual embodiments described and illustrated herein has discrete components and features that may be readily separated from or combined with the features of any of the other several embodiments without departing from the scope or spirit of the present disclosure.

Any recited method can be carried out in the order of events recited or any other order that is logically possible. Unless otherwise expressly stated, it is in no way intended that any method or aspect set forth herein be construed as requiring that its steps be performed in a specific order. Accordingly, where a method claim does not explicitly state in the claims or descriptions that the steps are to be limited to a particular order, it is in no way intended that an order be inferred in any respect. This holds for any possible non-express basis for interpretation, including logic concerning arrangement of steps or operational flow, meaning derived from grammatical organization or punctuation, or the number or type of aspects described in the specification.

All publications mentioned herein are incorporated by reference to disclose and describe the methods or materials in connection with which the publications are cited. The publications discussed herein are provided solely for their disclosure before the filing date of the present application. Nothing herein is to be construed as an admission that the present invention is not entitled to antedate such publication by prior invention. Further, the dates of publication provided herein can be different from the actual publication dates, which can require independent confirmation.

It is also to be understood that the terminology herein describes particular aspects only and is not intended to be limiting. Unless defined otherwise, all technical and scientific terms herein have the same meaning as commonly understood by one of ordinary skill in the art to which the disclosed compositions and methods belong. It can be further understood that terms, such as those defined in commonly used dictionaries, should be interpreted as having a meaning that is consistent with their meaning in the context of the specification and relevant art and should not be interpreted in an idealized or overly formal sense unless expressly defined herein.

Before describing the various aspects of the present disclosure, the following definitions are provided and should be used unless otherwise indicated. Additional terms may be defined elsewhere in the present disclosure.

### Definitions

As used herein, "comprising" is interpreted as specifying the presence of the stated features, integers, steps, or components but does not preclude the presence or addition of one or more features, integers, steps, components, or groups thereof. Moreover, each of the terms "by," "comprising," "comprises," "comprised of," "including," "includes," "included," "involving," "involves," "involved," and "such as" are used in their open, non-limiting sense and may be used interchangeably. Further, the term "comprising" is intended to include examples and aspects encompassed by the terms "consisting essentially of" and "consisting of." Similarly, "consisting essentially of" is intended to include examples encompassed by the term "consisting of."

As used in the specification and the appended claims, the singular forms "a," "an," and "the" include plural referents unless the context dictates otherwise. Thus, for example, reference to "a peptide," "a composition," or "a disorder" includes, but is not limited to, two or more such peptides, compositions, disorders, and the like.

Ratios, concentrations, amounts, and other numerical data can be expressed herein in a range format. Further, the endpoints of each of the ranges are significant both in relation to the other endpoint and independently of the other endpoint. There are many values disclosed herein, and each value is also disclosed as "about" that particular value in addition to the value itself. For example, if the value "10" is disclosed, then "about 10" is also disclosed. Ranges can be expressed herein as from "about" one particular value and to "about" another particular value. Similarly, when values are expressed as approximations, using the antecedent "about," the particular value forms a further aspect. For example, if the value "about 10" is disclosed, then "10" is also disclosed.

When a range is expressed, a further aspect includes from the one particular value and to the other particular value. For example, where the stated range includes one or both of the limits, ranges excluding either or both of those included limits are also included in the disclosure, e.g., the phrase "x to y" includes the range from `x' to 'y' as well as the range greater than `x' and less than 'y'. The range can also be expressed as an upper limit, e.g., 'about x, y, z, or less' and should be interpreted to include the specific ranges of `about x,' `about y,' and `about z' as well as the ranges of `less than x,' `less than y.' and 'less than z.' Likewise, the phrase `about x, y, z, or greater' should be interpreted to include the specific ranges of `about x,' 'about y,' and `about z' as well as the ranges of 'greater than x,' greater than y,' and 'greater than z.' In addition, the phrase "about `x' to 'y'," where 'x' and 'y' are numerical values, includes "about 'x' to about 'y'."

Such a range format is used for convenience and brevity and, thus, should be interpreted flexibly to include not only the numerical values explicitly recited as the limits of the range but also to include all the individual numerical values or sub-ranges encompassed within that range as if each numerical value and sub-range is explicitly recited. To illustrate, a numerical range of "about 0.1% to 5%" should be interpreted to include not only the explicitly recited values of about 0.1% to about 5% but also include individual values (e.g., about 1%, about 2%, about 3%, and about 4%) and the sub-ranges (e.g., about 0.5% to about 1.1%; about 5% to about 2.4%; about 0.5% to about 3.2%, and about 0.5% to about 4.4%, and other possible sub-ranges) within the indicated range.

As used herein, the terms "about," "approximate," "at or about," and "substantially" mean that the amount or value in question can be the exact value or a value that provides equivalent results or effects as recited in the claims or taught herein. That is, amounts, sizes, formulations, parameters, and other quantities and characteristics are not and need not be exact but may be approximate, larger or smaller, as desired, reflecting tolerances, conversion factors, rounding, measurement error, and the like, and other factors known to those of skill in the art such that equivalent results or effects are obtained. In some circumstances, the value that provides equivalent results or effects cannot be reasonably determined. In such cases, as used herein, "about" and "at or about" mean the nominal value indicated ±10% variation unless otherwise indicated or inferred. In general, an amount, size, formulation, parameter, or other quantity or characteristic is "about," "approximate," or "at or about," whether or not expressly stated to be such. Where "about," "approximate," or "at or about" is used before a quantitative value, the parameter also includes the specific quantitative value itself unless expressly stated otherwise.

As used herein, the term "therapeutically effective amount" refers to an amount sufficient to achieve the desired therapeutic result or to have an effect on undesired symptoms but generally insufficient to cause adverse side effects. The specific therapeutically effective dose level for any particular patient will depend upon a variety of factors, including the disorder being treated and the severity of the disorder; the specific composition employed; the age, body weight, general health, sex, and diet of the patient; the time of administration; the route of administration; the rate of excretion of the specific compound employed; the duration of the treatment; drugs used in combination or coincidental with the particular compound employed and like factors within the knowledge and expertise of the health practitioner and which may be well known in the medical arts. In the case of treating a particular disease or condition, in some instances, the desired response can be inhibiting the progression of the disease or condition. This may involve only slowing the progression of the disease temporarily. However, in other instances, it may be desirable to permanently halt the progression of the disease. This can be monitored by routine diagnostic methods known to one of ordinary skill in the art for any particular disease. The desired response to treatment of the disease or condition can also be delaying the onset or even preventing the onset.

For example, it is well within the skill of the art to start doses of a compound at levels lower than those required to achieve the desired therapeutic effect and to increase the dosage gradually until the desired effect is achieved. If desired, the effective daily dose can be divided into multiple doses for administration. Consequently, single-dose compositions can contain such amounts or submultiples thereof to make up the daily dose. The individual physician can adjust the dosage in the event of any contraindications. It is generally preferred that a maximum dose of the pharmacological agents of the invention (alone or in combination with other therapeutic agents) be used, that is, the highest safe dose according to sound medical judgment. However, a patient may insist on a lower or tolerable dose for medical reasons, psychological reasons, or virtually any other reason.

A response to a therapeutically effective dose of a disclosed compound or composition can be measured by determining the physiological effects of the treatment or medication, such as the decrease or lack of disease symptoms following the administration of the treatment or pharmacological agent. Other assays will be known to one of ordinary skill in the art and can be employed for measuring the level of the response. The amount of a treatment may be varied, for example, by increasing or decreasing the amount of a disclosed compound or pharmaceutical composition, changing the disclosed compound or pharmaceutical composition administered, changing the route of administration, changing the dosage timing, and so on. Dosage can vary and can be administered in one or more dose administrations daily for one or several days. Guidance can be found in the literature for appropriate dosages for given classes of pharmaceutical products.

As used herein, "optional" or "optionally" means that the subsequently described event or circumstance can or cannot occur. The description includes instances where said event or circumstance occurs and those where it does not.

As used interchangeably herein, "subject," "individual," or "patient" can refer to a vertebrate organism, such as a mammal (e.g., human). "Subject" can also refer to a cell, a population of cells, a tissue, an organ, or an organism, preferably to a human and constituents thereof.

As used herein, "treating" and "treatment" generally refer to obtaining a desired pharmacological or physiological effect. The effect can be but does not necessarily have to be prophylactic in preventing or partially preventing a disease, symptom, or condition such as Parkinson's disease. The effect can be therapeutic regarding a partial or complete cure of a disease, condition, symptom, or adverse effect attributed to the disease, disorder, or condition. The term "treatment" as used herein can include any treatment of a disorder in a subject, particularly a human. It can include any one or more of the following: (a) preventing the disease from occurring in a subject who may be predisposed to the disease but has not yet been diagnosed as having it; (b) inhibiting the disease, i.e., arresting its development; and (c) relieving the disease, i.e., mitigating or ameliorating the disease or its symptoms or conditions. The term "treatment," as used herein, can refer to both therapeutic treatment alone, prophylactic treatment alone, or both therapeutic and prophylactic treatment. Those in need of treatment (i.e., subjects in need thereof) can include those already with the disorder or those in which the disorder is to be prevented. As used herein, the term "treating" can include inhibiting the disease, disorder, or condition, e.g., impeding its progress, and relieving the disease, disorder, or condition, e.g., causing regression of the disease, disorder, or condition. Treating the disease, disorder, or condition can include ameliorating at least one symptom of the particular disease, disorder, or condition, even if the underlying pathophysiology is not affected, e.g., such as treating the pain of a subject by administration of an analgesic agent even though such agent does not treat the cause of the pain.

As used herein, "dose," "unit dose," or "dosage" can refer to physically discrete units suitable for use in a subject, each unit containing a predetermined quantity of a disclosed compound or a pharmaceutical composition thereof calculated to produce the desired response or responses in association with its administration.

As used herein, "therapeutic" can refer to treating, healing, or ameliorating a disease, disorder, condition, or side effect or decreasing the rate of advancement of a disease, disorder, condition, or side effect.

"Amino acid," as used herein, refers to a molecule containing both an amino group and a carboxyl group. Amino acids include α-amino acids and β-amino acids. In certain forms, an amino acid is an alpha amino acid. Amino acids can be natural or synthetic. Amino acids include but are not limited to, the twenty standard or canonical amino acids: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trk, W), Tyrosine (Tyr, Y), and Valine (Val, V). Common non-standard or non-canonical amino acids include but are not limited to, selenocysteine, pyrrolysine, and N-formylmethionine. The term "synthetic amino acid" or "non-natural amino acid," as used herein, refers to an organic compound with a structure similar to a natural amino acid so that it mimics the structure and reactivity of a natural amino acid. The synthetic amino acid, as defined herein, generally increases or enhances the properties of a peptide (e.g., selectivity or stability) when the synthetic amino acid is either substituted for a natural amino acid or incorporated into a peptide.

The terms "peptide," "protein," "polypeptide," or "polyamino acid" are used interchangeably to refer to a natural or synthetic molecule comprising two or more amino acids linked by the carboxyl group of one amino acid to the amino group of another. In addition, as used herein, the term "polypeptide" refers to amino acids joined to each other by peptide bonds or modified peptide bonds, e.g., peptide isosteres, etc., and may contain modified amino acids other than the 20 gene-encoded amino acids. The polypeptides can be modified by either natural processes, such as post-translation processing, or by chemical modification techniques which are well-known in the art. Modifications can occur anywhere in the polypeptide, including the peptide backbone, the amino acid side-chains, and the amino or carboxyl termini. The same type of modification can be present in the same or varying degrees at several sites in the given polypeptide. Also, a given polypeptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleoside or nucleotide derivative, covalent attachment of a lipid or lipid derivative, covalent attachment of a phosphatidylinositol, disulfide bond formation, demethylation, formation of cysteine or pyroglutamate, formylation, gamma-carboxylation, glycosylation, GPI anchor formation, hydroxylating, iodination, methylation, myristoylation, oxidation, PEGylation, proteolytic processing, phosphorylation, prenylation, racemization, selenoylation, sulfation, and transfer-RNA mediated addition of amino acids to proteins such as arginylation. Also included in the term "polypeptides" are cis- and trans-isomers, R- and S-enantiomers, D-isomers, L-isomers, diastereomers, conformers, and mixtures thereof.

The term "residue," as used herein, refers to an amino acid that is incorporated into a polypeptide. The amino acid may be a naturally occurring amino acid and, unless otherwise limited, may encompass analogs of natural amino acids that can function in a similar manner as naturally occurring amino acids.

A "variant," as used herein, means a polypeptide comprising one or more modifications such as substitutions, deletions, and/or truncations of one or more specific amino acid residues in the corresponding wild-type peptide. A variant of a polypeptide may be naturally occurring or synthetic and may have 50%, 55%, 60%, 65%, 70%, 75%, 80%, 85%, 90%, 95%, 96%, 97%, 98% or 99% identity with the wild-type polypeptide.

Conservative substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Exemplary conservative amino acids substitution groups are: valine-leucine-isoleucine, phenylalanine-tyrosine, lysine-arginine, alanine-valine, aspartic acid-glutamic acid, and asparagine-glutamine. Further exemplary conservative substitutions are provided in the below table, with others being known in the art:

### Amino Acid Substitutions

| Original Residue | Exemplary Conservative Substitutions |
|---|---|
| Ala | Ser |
| Arg | Lys; Gln |
| Asn | Gln; His |
| Asp | Glu |
| Cys | Ser |
| Gin | Asn, Lys |
| Glu | Asp |
| Gly | Pro |
| His | Asn;Gln |
| Ile | Leu; Val |
| Leu | Ile; Val |
| Lys | Arg; Gln |
| Met | Leu; Ile |
| Phe | Met; Leu; Tyr |
| Ser | Thr |
| Thr | Ser |
| Trp | Tyr |
| Tyr | Trp; Phe |

The term "inhibit" refers to a decrease in an activity, response, condition, disease, or other biological parameter. This can include but is not limited to, the complete ablation of the activity, response, condition, or disease. This may also include, for example, a 10% reduction in the activity, response, condition, or disease as compared to the native or control level. Thus, the reduction can be a 10%, 20%, 30%, 40%, 50%, 60%, 70%, 80%, 90%, 100%, or any amount of reduction in between as compared to native or control levels.

### Peptides

Non-natural, synthetic polypeptides are disclosed that contain a chemically stabilized α-helical shape that mimics domains of FADD that facilitate its interaction with LRRK2, allowing the polypeptides to bind to LRRK2 in physiological or supraphysiological conditions and diminish these interactions which play a role in various medical disorders and conditions, for example, such as Parkinson's disease.

A strategic method to bestow drug-like properties onto α-helical peptides was developed called peptide "stapling" (see Schafmeister, C. E. et al. J. Am. Chem. Soc. 2000 122(24):5891-2). This strategy involves the incorporation of two non-natural amino acids within the peptide sequence that are disubstituted to contain α-methyl and α-alkenyl groups. The peptide secondary structure is conformationally locked via, e.g., a Grubbs I catalyzed ring-closing metathesis reaction to form a macrocyclic ring using the α-alkenyl groups (see Mansuy D. et al. Med Sci (Paris). 2005 21(11):995-6). Further studies have shown that this chemical modification introduces an entropically favorable pre-ordered binding state that increases substrate binding affinity, causes resistance to proteolytic degradation, and greatly enhances cell permeability (see Manschwetus, J. T. et al. Molecules 2019, 24(8):E1567; Flaherty, B. R. et al. ACS Infect Dis 2019, 5(4):506-514; Fulton, M. D. et al. Bioorg Med Chem 2018, 26(6):1167-1173; Teng, Y. et al. Cancer Res 2016, 76(17):5133-42; Wang, Y. et al. ACS Chem Biol 2015, 10(6):1502-10; and Wang, Y. et al. ACS Chem Biol 2014, 9(3):635-642). By applying this chemical modification to a peptide-based scaffold, large binding areas on protein surfaces can be targeted with a high degree of specificity that would otherwise be elusive for targeting using a small molecule approach.

"Peptide stapling" is a term coined from a synthetic methodology wherein two olefin-containing side-chains present in a polypeptide chain are covalently joined (e.g., "stapled together") using a ring-closing metathesis (RCM) reaction to form a cross-linked ring (see, the cover art for J. Org. Chem. 2001 66(16) describing metathesis-based crosslinking of alpha-helical peptides; Blackwell et al. Angew. Chem. Int. Ed. 1994 37:3281). However, the term "peptide stapling" as used herein encompasses the joining of two double bond-containing side chains, two triple bond-containing side chains, or one double bond-containing and one triple bond-containing side chain, which may be present in a polypeptide chain, using any number of reaction conditions and/or catalysts to facilitate such a reaction, to provide a single "staple" polypeptide. Additionally, the term "peptide stitching," as used herein, refers to multiple and tandem "stapling" events in a single polypeptide chain to provide a "stitched" (multiply stapled) polypeptide. The disclosed polypeptides can contain a hydrocarbon staple to chemically stabilize an α-helical shape.

In some aspects, the disclosed peptides include a hydrocarbon staple. The genesis of the hydrocarbon stapling technique can be traced to the ruthenium-based Grubb's catalyst used for ring-closing metathesis. The α-helix features 3.6 residues per complete turn, which places the i, i+4, i+7, and i+11 side chains on the same face of the folded structure. Therefore, stapling cross-links two α,α disubstituted amino acids bearing olefinic chains of variable length at positions "i" and "i+4" or "i+7" in the peptide sequence. In general, the first step in designing stapled peptides for macromolecular targets is the identification of appropriate sites for incorporating the non-natural amino acids used from the hydrocarbon cross-link. Generally, residues that are not involved in the target recognition are chosen as potential sites for incorporation of olefin-bearing building blocks. These sites are subsequently used to incorporate various suitable stapling systems such as i and i+3, i and i+4, or i and i+7. The classical strategy to stabilize the α-helical conformation in peptides employs covalent bonds between the i and i+3, i and i+4, or i and i+7 side chain groups.

In some aspects, the polypeptide comprises two non-natural amino acids on the same side of the α-helix that are crosslinked to stabilize the α-helical shape. For example, two non-natural amino acids can be four (i and i+4) or seven (i and i+7) amino acids apart. In some cases, the non-natural amino acids can comprise olefinic side chains, such as (S)-2-(2'-propenyl)alanine ("S3"); (S)-2-(4'-pentenyl)alanine ("S5"); (S)-2-(5'-hexenyl)alanine ("S6"); (S)-2-(7'-octenyl)alanine ("S8"); (R)-2-(2'-propenyl)alanine ("R3"); (R)-2-(4'-pentenyl)alanine ("R5"); (R)-2-(5'-hexenyl)alanine ("R6"); and (S)-2-(7'-octenyl)alanine ("S8").

The disclosed peptides can be stapled in any suitable pairing, including, but not limited to, a pairing selected from the group consisting of an S5-S5 pairing (i.e., i and i+4), an S5-R8 pairing (i.e., i and i+7), an S8-R5 pairing (i.e., i and i+7), an R3-S6 pairing (i.e., i and i+3), an R6-S3 pairing (i.e., i and i+3), an R3-S5 pairing (i.e., i and i+3), an R5-S3 pairing (i.e., i and i+3), or combinations of pairings within the polypeptide sequence.

The hydrocarbon bridge can then be formed, for example, by a ring-closing metathesis reaction catalyzed by benzylidenebis(tricyclohexyl-phosphine)-dichlororuthenium (Grubb's catalyst). In other aspects, the ring-closing metathesis reaction can be performed by any other suitable metathesis catalyst as would be available to a person of ordinary skill in the art.

Stapling a peptide using an all-hydrocarbon cross-link has been shown to help maintain its native conformation and/or secondary structure, particularly under physiologically relevant conditions. For example, stapling a polypeptide predisposed to having an α-helical secondary structure can constrain the polypeptide to its native α-helical conformation. The constrained secondary structure may, for example, increase the peptide's resistance to proteolytic cleavage, may increase the peptide's hydrophobicity, may allow for better penetration of the peptide into the target cell's membrane (e.g., through an energy-dependent transport mechanism such as pinocytosis), and/or may lead to an improvement in the peptide's biological activity relative to the corresponding uncrosslinked (e.g., "unstapled") peptide.

A number of alternative stapling methods are known to those in the art, each using a different form of macrocyclization chemistry and giving rise to stapled peptides with different bioactive properties. For example, the stapling may be one-component stapling. One-component stapling involves a direct bond-forming reaction between the side-chains of two amino acids. In some aspects, the one-component stapling method may comprise formation of an amide bond between two side chains of amino acids in the peptide. In some aspects, the one-component stapling technique may comprise, for example, a ring-closing metathesis, a lactamization, a cycloaddition (such as the Cu(I)-catalyzed azide-alkyne cycloaddition (CuAAC, "copper-catalyzed click reaction") or ring-strained azide-alkyne cycloaddition), a reversible reaction (such as formation of a disulfide bride or an oxime linkage), or thioether formation. The stapling technique may alternatively be a two-component stapling. Two-component stapling involves a bifunctional linker compound that forms a staple by reacting with two complementary native or non-native amino acids in the peptide of interest. Two-component stapling may employ, for example, a photoswitchable linker or a functionalized "double click" linker. In some aspects, the precursors may independently comprise residues that are an amino acid analog having an alkyne group on the side chain or an amino acid having an azide group on the side chain, and these groups react with a precursor to the staple having complementary alkyne and/or azide groups to form a triazole. Additional examples of staples and stapling methods appropriate for use in the stapled peptides disclosed herein are described in Walensky, L.D. et al., J. Med. Chem. 2014, 57:6275-6288; Lau, Y. H. et al., Chem. Soc. Rev. 2014, 00:1-12; Joy, S.T. et al., Chem. Commun. 52(33):5738-5741; and Zhao, H. et al. Angew. Chem. Int. Ed. 2016, 55:12088-12093, each of which are incorporated herein by reference in their entirety.

Other forms of chemical stabilization may also be used in the disclosed peptides. For example, amino acids and unstapled, partially stapled, and stapled peptides and proteins, and unstitched, partially stitched, and stitched peptides and proteins may exist in particular geometric or stereoisomeric forms. The disclosed peptides can include all such compounds, including cis- and trans-isomers, R- and S-enantiomers, diastereomers, (D)-isomers, (L)-isomers, racemic mixtures thereof, and other mixtures thereof. Where an isomer-enantiomer is preferred, it may, in some aspects, be provided substantially free of the corresponding enantiomer and may also be referred to as "optically enriched." "Optically enriched," as used herein, means that the compound is made up of a significantly greater proportion of one enantiomer. In certain aspects, the compound of the present invention is made up of at least about 90% by weight of a preferred enantiomer. In other aspects, the compound is made up of at least about 95%, 98%, or 99% or more by weight of a preferred enantiomer.

The polypeptide can be a synthetic peptide containing non-natural amino acids or a peptidomimetic. As used herein, "peptidomimetic" means a mimetic of a peptide which includes some alteration of the normal peptide chemistry. Peptidomimetics typically enhance some properties of the original peptide, such as increased stability, increased efficacy, enhanced delivery, increased half-life, etc. Use of peptidomimetics can involve the incorporation of a non-amino acid residue with non-amide linkages at a given position. One aspect of the present invention is a peptidomimetic wherein the compound has a bond, a peptide backbone, or an amino acid component replaced with a suitable mimic. Some non-limiting examples of non-natural amino acids which may be suitable amino acid mimics include, but are not limited to, β-alanine, L-α-aminobutyric acid, L-γ-aminobutyric acid, L-α-aminoisobutyric acid, L-ε-aminocaproic acid, 7-aminoheptanoic acid, L-aspartic acid, L-glutamic acid, N-ε-Boc-N-α-CBZ-L-lysine, N-ε-Boc-N-α-Fmoc-L-lysine, L-methionine sulfone, L-norleucine, L-norvaline, N-α-Boc-N-δ-Cbz-L-ornithine, N-δ-Boc-N-α-Cbz-L-orinithine, Boc-p-nitro-L-phenylalanine, Boc-hydroxyproline, and Boc-L-thioproline.

The disclosed peptides may also be substituted with any number of substituents or functional moieties. In general, the term "substituted" refers to the replacement of a hydrogen group in a given structure with a specified substituent group. When more than one position in any given structure may be substituted with more than one substituent selected from a specified group, the substituent may be either the same or different at every position. As used herein, the term "substituted" is contemplated to include substitution with all permissible substituents of organic compounds, any of the substituents described herein (for example, aliphatic, alkyl, alkenyl, alkynyl, heteroaliphatic, heterocyclic, aryl, heteroaryl, acyl, oxo, imino, thioxo, cyano, isocyano, amino, azido, nitro, hydroxyl, thio, halo, etc.), and any combination thereof (for example, aliphatic amino, heteroaliphaticamino, alkylamino, heteroalkylamino, arylamino, heteroarylamino, alkylaryl, arylalkyl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, acyloxy, and the like) that results in the formation of a stable moiety. The disclosed peptides can contain any and all such combinations in order to arrive at a stable substituent/moiety. For the disclosed peptides, heteroatoms such as nitrogen may have hydrogen substituents and/or any suitable substituent as described herein, which satisfy the valencies of the heteroatoms and result in the formation of a stable moiety.

Peptides and peptidomimetics can be prepared by any method, such as by synthesizing the peptide or peptidomimetic or by expressing a nucleic acid encoding an appropriate amino acid sequence in a cell and harvesting the peptide from the cell. Of course, a combination of such methods also can be used.

Examples of chemical synthesis technologies are solid phase synthesis and liquid phase synthesis. Solid phase synthesis methods are largely classified by the tBoc method and the Fmoc method, depending on the type of protective group used. Typically used protective groups include tBoc (t-butoxycarbonyl), Cl--Z (2-chlorobenzyloxycarbonl), Br-Z (2-bromobenzyloxycarbonyl), Bzl (benzyl), Fmoc (9-fluorenylmethoxycarbonyl), Mbh (4,4'-dimethoxydibenzyhydryl), Mtr (4-methoxy-2,3,6-trimethylbenzenesulfonyl), Trt (trityl), Tos (tosyl), Z (Benzyloxycarbonyl), and Clz-Bzl (2,6-dichlrobenzyl) for the amino groups; NO2 (nitro) and Pmc (2,2,5,7,8-pentamethylchromane-6-sulfonyl) for the guanidino groups; and t-Bu (t-butyl) for the hydroxyl groups. After synthesis of the desired peptide, it is subjected to one or more deprotection reactions and cut out from the solid support. Such peptide cutting reactions may be carried out with hydrogen fluoride or trifluoromethane sulfonic acid for the Boc method or with TFA for the Fmoc method. Methods of de novo synthesizing of peptides and peptidomimetics are described, for example, in Chan et al., Fmoc Solid Phase Peptide Synthesis, Oxford University Press, Oxford, United Kingdom, 2005; and Peptide and Protein Drug Analysis, ed. Redi., R., Marcel Dekker, Inc., 2000.

Alternatively, the peptide may be synthesized using recombinant techniques. In this case, a nucleic acid encoding the peptide is cloned into an expression vector under the control of expression control sequences (e.g., a promoter, a terminator, and/or an enhancer), allowing its expression. The expression vector is then transfected into a host cell (e.g., a human, CHO, mouse, monkey, fungal or bacterial host cell), and the transfected host cell is cultivated under conditions suitable for the expression of the peptide. Standard recombinant DNA and molecular cloning techniques are described, for example, in Sambrook and Maniatis, Molecular Cloning: A Laboratory Manual, Second Edition, Cold Spring Harbor Laboratory Press, Cold Spring Harbor, N.Y. (1989); Silhavy et al., Experiments with Gene Fusions, Cold Spring Harbor Laboratory Press, Cold Spring Harbor, N.Y. (1984); and Ausubel et al., Current Protocols in Molecular Biology, published by Greene Publishing Assoc. and Wiley-Interscience (1987).

The method of producing the peptide may optionally comprise the steps of purifying said peptide, chemically modifying said peptide, and/or formulating said peptide into a pharmaceutical composition.

In some aspects, the stapled peptide includes a helical motif (i.e., a stapled helical peptide). Different amino acid residues have different propensities for forming different secondary structures. For example, methionine (M), alanine (A), leucine (L), glutamate (E), and lysine (K) all have especially high α-helix forming propensities. Thus in some aspects, the stapled polypeptide includes one or more amino acid residues selected from methionine (M), alanine (A), leucine (L), glutamate (E), and lysine (K). In contrast, proline (P) and glycine (G) are α-helix disruptors. Thus, in some aspects, the stapled polypeptide does not include one or more proline (P) and glycine (G) amino acid residues.

In some aspects, the polypeptide mimics the death domain of FADD, for example, the residues 173 to 190 of FADD (UniProtKB - Q6LCG1). In some aspects, the polypeptide mimics the α-helix forming peptide having the sequence: KVADLVQEVQQARDLQNRSK (SEQ ID NO. 1). For example, the polypeptide can comprise a variant of the amino acid sequence SEQ ID NO. 1 wherein the variant comprises at least one pair of non-natural amino acids inserted into the amino acid sequence that are cross-linked to stabilize the α-helical shape. In some aspects, the polypeptide can comprise a variant of the amino acid sequence SEQ ID NO. 1 wherein the variant comprises at least one pair of olefin-terminated, non-natural amino acids (for example, selected from (S)-2-(4'-pentenyl)alanine, (R)-2-(4'-pentenyl)alanine, (S)-2-(7'-octenyl)alanine, (R)-2-(7'-octenyl)alanine, or variants thereof) that form a hydrocarbon staple to stabilize the α-helical shape.

In some aspects, the polypeptide is formed from a peptide comprising an amino acid sequence having at least 80% sequence similarity to a sequence selected from SEQ ID NO. 2 to SEQ ID NO. 6:

| | |
|---|---|
| KXADLXQEVQQARDLQNRSK | (SEQ ID NO. 2); |
| KVADLXQEVXQARDLQNRSK | (SEQ ID NO. 3); |
| KVADLVQEXQQAXDLQNRSK | (SEQ ID NO. 4); |
| KVADLVQEVQQXRDLXNRSK | (SEQ ID NO. 5); and |
| KVADLVQEVQQAXDLQXRSK | (SEQ ID NO. 6; |

wherein X is (S)-2-(4'-pentenyl)alanine.

In some aspects, the polypeptide is formed from a peptide comprising an amino acid sequence having at least 85%, at least 90%, at least 95%, or at least 99% sequence similarity to a sequence selected from SEQ ID NO. 2, SEQ ID NO. 3, SEQ ID NO. 4, SEQ ID NO. 5, and SEQ ID NO. 6. In some aspects, the polypeptide is formed from a peptide comprising an amino acid sequence selected from SEQ ID NO. 2, SEQ ID NO. 3, SEQ ID NO. 4, SEQ ID NO. 5, and SEQ ID NO. 6.

Representative examples of stapled peptides derived from the death domain of FADD include: 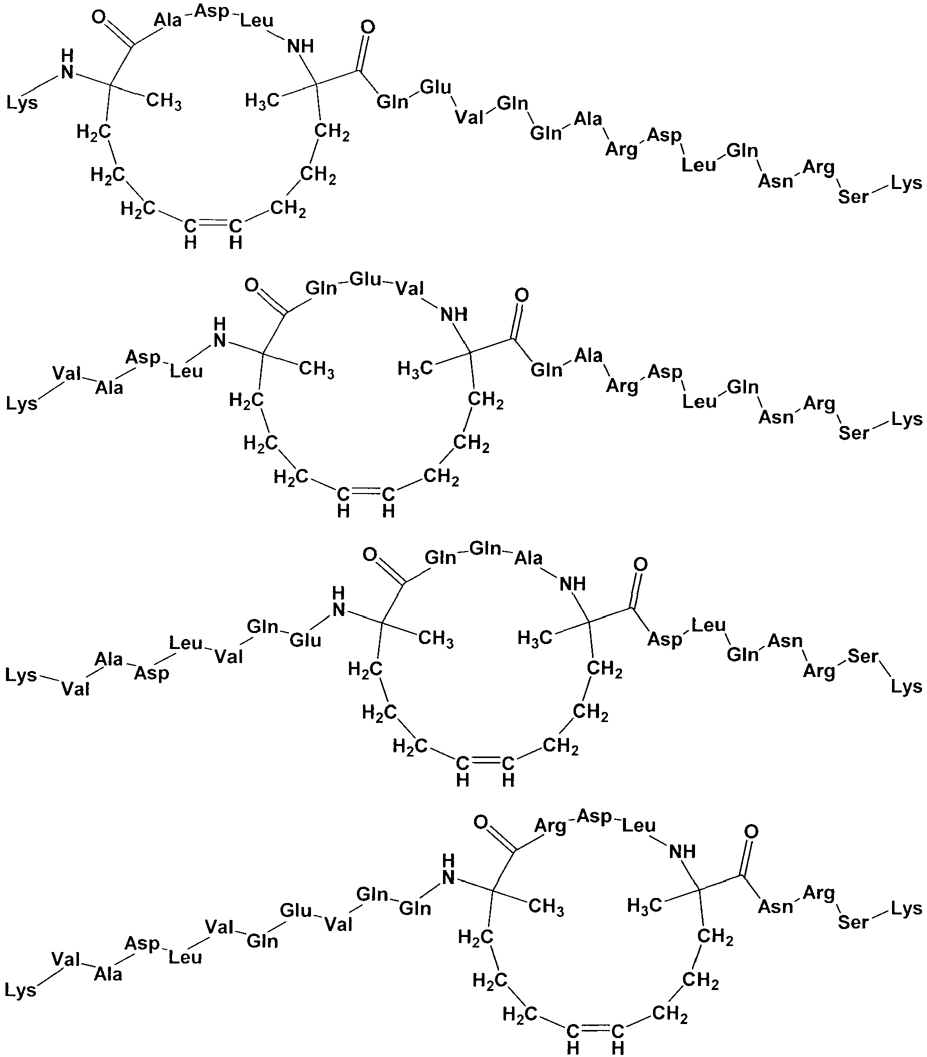 and 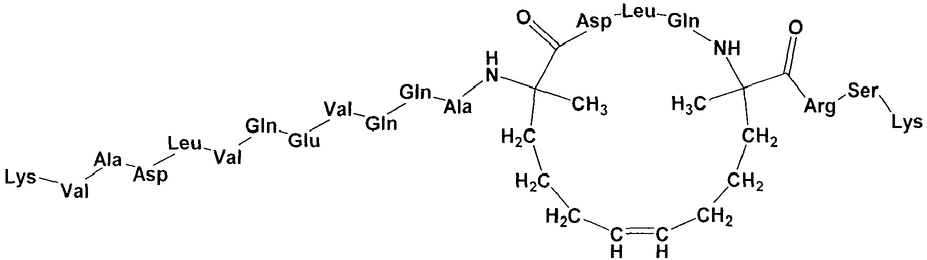 wherein
Ala is an alanine residue or a conservative substitution or a derivative thereof;
Arg is an arginine residue or a conservative substitution or a derivative thereof;
Asn is an asparagine residue or a conservative substitution or a derivative thereof;
Asp is an aspartate residue or a conservative substitution or a derivative thereof;
Gln is a glutamine residue or a conservative substitution or a derivative thereof;
Glu is a glutamate residue or a conservative substitution or a derivative thereof;
Leu is a leucine residue or a conservative substitution or derivative thereof;
Lys is a lysine residue or a conservative substitution or a derivative thereof;
Ser is a serine residue or a conservative substitution or a derivative thereof; and
Val is a valine residue or a conservative substitution or a derivative thereof.

In some aspects, the peptide is about 5 to 100 amino acids in length, including about 5 to 50 amino acids in length. In some aspects, the peptide is less than 51 amino acids in length, including less than 50, 45, 40, 35, 30, 25, 20, 15, or 10 amino acids in length. Therefore, the provided polypeptide can further constitute a fusion protein or otherwise have additional N-terminal, C-terminal, or intermediate amino acid sequences.

In some aspects, introduction of a hydrocarbon staple results in poor water solubility and cell permeability. To increase cell permeability and solubility of these peptides, the disclosed polypeptide can be linked to a cell permeability moiety. A "cell permeability" or a "cell-penetration" moiety refers to any molecule known in the art which is able to facilitate or enhance penetration of molecules through membranes. Non-limiting examples include: hydrophobic moieties such as lipids, fatty acids, steroids, and bulky aromatic or aliphatic compounds; moieties which may cell-membrane receptors or carriers, such as steroids, vitamins and sugars, natural and non-natural amino acids and transporter peptides. Examples for lipidic moieties which may be used according to the present invention include: Lipofectamine, TransfectACE, Trasfectam, Cytofectic, DMRIE, DLRIE, GAP-DLRIE, DOTAP, DOPE, DMEAP, DODMP, DPOC, DDAB, DOSPA, EDLPC, EDMPC, DPH, TMADPH, CTAB, lysyl-PE, DC-Cho, -alanyl cholesterol, DCGS, DPPES, DCPE, DMAP, DMPE, DOGS, DOHME, DPEPC, Pluronic, Tween, BRIJ, plasmalogen, phosphatidylethanolamine, phosphatidylcholine, glycerol-3-ethylphosphatidylcholine, dimethyl ammonium propane, trimethyl ammonium propane, diethyl ammonium propane, triethylammonium propane, dimethyldioctadecylammonium bromide, a sphingolipid, sphingomyelin, a lysolipid, a glycolipid, a sulfatide, a glycosphingolipid, cholesterol, cholesterol ester, cholesterol salt, N-succinyldioleoylphosphatidylethanoleamine. 1,2-dioleoyl-sn-glycerol, 1,3-dipalmitoyl-2-succinylglycerol, 1,2-dipalmatoyl-sn-3-succinylglycerol, 1-hexadecyl-2-palmitoylglycerophosphatidylethanolamine, palmitoylhomocysteine, N,N'-bis(dodecylaminocarbonylmethylene)-N,N'-bis(N,N,N-trimethylammoniumethylaminocarbonylmethylene)ethylene diamine tetraiodide; N5,N"-bis(hexadecylaminocarbonylmethylene)-N,N',N"-tris(N,N,N-trimethylammoniumetyhlaminocarbonylmetylene)diethyltriamine hexaiodide; N,N-bis(dodecylaminocarbonylmethylene-N,N-bis(N,N,N-trimethylammoniumethylaminocarbonylmethylene)cyclohexene-1,4-diamine tetraiodide; 1,7,7-tetra(N,N,N,N-tetramethylammoniumethylaminocarbonylmethylene)-3-hexadecylaminocarbonylmethylene-1,3,7-triazaheptane heptaiodide; N5,N5,N',N'-tetra(N,N,-trimethylammoniumethylaminocarbonylmethylene)-N'-(15,2-dioleoylglycero-3-phosphoethanolaminocarbonylmethylene)diethylene triamine tetraiodide; dioleolylphosphatidylethanolamine; a fatty acid, a lysolipid, phosphatidylcholine, phosphatidylethanolamine, phosphatidylserine, phosphatidylglycerol, phosphatidylinositol, a sphingolipid, a glycolipid, a glucolipid, a sulfatide, a glycosphingolipid, phosphatidic acid, palmitic acid, stearic acid, arachidonic acid, oleic acid, a lipid bearing a polymer, a lipid bearing a sulfonated saccharide, cholesterol, tocopherol hemisuccinate, a lipid with an ether-linked fatty acid, a lipid with an ester-linked fatty acid, a polymerized lipid, diacetyl phosphate, stearylamine, cardiolipin, a phospholipid with a fatty acid of 6 to 8 carbons in length, a phospholipid with asymmetric acyl chains, 6-(5-cholesten-3b-yloxy)-1-thio-b-D-galactopyranoside, digalactosylglyceride, 6-(5-cholesten-3b-yloxy)hexyl-6-amino-6-deoxy-1-thio-b-D-galactopyranoside, 6-(5-cholesten-3b-yloxy)hexyl-6-amino-6-deoxyl-1-thio-a-D-mannopyranoside, 12(((7'-diethylamino-coumarin-3-yl)carbonyl)methylamino)-octadecanoic acid; N-[12-(((T -diethylaminocoumarin-3-yl)carbonyl)methylamino)octadecanoyl]-2-aminopalmitic acid, (cholesteryl)-4'-trimethylammonio)butanoate; N-succinyldioleoyl-phosphatidylethanoleamine; 1,2-dioleoyl-sn-glycerol; 1'-dipalmitoyl-sn-S-succinyl-glycerol, 1,3-dipalmitoyl-2-succinylglycerol, 1-hexadecyl-2-palmitoylglycero-phosphoethanolamine, and palmitoylhomocysteine.

In some aspects, the disclosed polypeptide can be linked to a protein transduction domain to effectively enter a cell. The protein transduction domain sequence can be any internalization sequence known or newly discovered in the art or conservative variants thereof. Non-limiting examples of cellular internalization transporters and sequences include polyarginine (e.g., R⁹), Antennapedia sequences, TAT, HIV-TAT, Pentratin, Antp-3A (Antp mutant), Buforin II, Transportan, MAP (model amphipathic peptide), K-FGF, Ku70, Prion, pVEC, Pep-1, SynB1, Pep-7, HN-1, BGSC (Bis-Guanidinium-Spermidine-Cholestero), and BGTG (Bis-Guanidinium-Tren-Cholesterol).

Addition of water soluble polymers or carbohydrates to polypeptide drugs has been shown to prevent their degradation and increase their half-life. For instance, "PEGylation" of polypeptide drugs protects them and improves their pharmacodynamic and pharmacokinetic profiles. The PEGylation process attaches repeating units of polyethylene glycol (PEG) to a polypeptide drug. PEGylation of molecules can lead to increased resistance of drugs to enzymatic degradation, increased half-life in vivo, reduced dosing frequency, decreased immunogenicity, increased physical and thermal stability, increased solubility, increased liquid stability, and reduced aggregation. Therefore, in some aspects, the disclosed polypeptide is covalently linked to a water soluble polymer, such as polyethylene glycol.

The most common route for PEG conjugation of polypeptides has been to activate the PEG with functional groups suitable for reactions with lysine and N-terminal amino acid groups. The monofunctionality of methoxyPET makes it particularly suitable for protein and peptide modification because it yields reactive PEGs that do not produce cross-linked polypeptides as long as diol PEG has been removed. Branched structures of PEG have also been proven to be useful for PEGylation of a protein or a peptide. For example, a branched PEG attached to a protein has properties of a much larger molecule than a corresponding linear mPEG of the same molecular weight. Branched PEGs also have the advantage of adding two PEG chains per attachment site on the protein, therefore reducing the chance of protein inactivation due to attachment. Furthermore, these structures are more effective in protecting proteins from proteolysis, in reducing antigenicity, and in reducing immunogenicity.

To increase cell permeability and solubility of these peptides, the peptides can be optimized to increase their amphipathic properties. In some cases, an overall net charge (neutral or positive) is needed for permeability. Any method that alters the overall net charge can affect permeability. In some cases, 1, 2, 3, 4, or more hydrophilic residues can be added on the solvent-exposed face of the helix. For example, the hydrophilic residue can be a lysine, aspartic acid, glutamic acid, arginine, histidine, serine, asparagine, or glutamine. In some cases, lysine and/or arginine are used since they have positive charges that help increase permeability. Non-natural amino acids bearing hydrophilic or charged properties can also be added.

### Methods of Making

The polypeptides described herein can be prepared in a variety of ways known to one skilled in the art of organic synthesis or variations thereon as appreciated by those skilled in the art. The compounds described herein can be prepared from readily available starting materials. Optimum reaction conditions can vary with the particular reactants or solvents used, but such conditions can be determined by one skilled in the art.

Variations on the compounds described herein include the addition, subtraction, or movement of the various constituents as described for each compound. Similarly, when one or more chiral centers are present in a molecule, the chirality of the molecule can be changed. Additionally, compound synthesis can involve the protection and deprotection of various chemical groups. The use of protection and deprotection, and the selection of appropriate protecting groups, can be determined by one skilled in the art. The chemistry of protecting groups can be found, for example, in Wuts and Greene, Protective Groups in Organic Synthesis, 4th Ed., Wiley & Sons, 2006, which is incorporated herein by reference in its entirety.

The starting materials and reagents used in preparing the disclosed compounds and compositions are either available from commercial suppliers such as Aldrich Chemical Co., (Milwaukee, WI), Acros Organics (Morris Plains, NJ), Fisher Scientific (Pittsburgh, PA), Sigma (St. Louis, MO), Pfizer (New York, NY), GlaxoSmithKline (Raleigh, NC), Merck (Whitehouse Station, NJ), Johnson & Johnson (New Brunswick, NJ), Aventis (Bridgewater, NJ), AstraZeneca (Wilmington, DE), Novartis (Basel, Switzerland), Wyeth (Madison, NJ), Bristol-Myers-Squibb (New York, NY), Roche (Basel, Switzerland), Lilly (Indianapolis, IN), Abbott (Abbott Park, IL), Schering Plough (Kenilworth, NJ), or Boehringer Ingelheim (Ingelheim, Germany), or are prepared by methods known to those skilled in the art following procedures set forth in the references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry (John Wiley and Sons, 4th Edition); and Larock's Comprehensive Organic Transformations (VCH Publishers Inc., 1989). Other materials, such as the pharmaceutical carriers disclosed herein, can be obtained from commercial sources.

Reactions to produce the compounds described herein can be carried out in solvents, which can be selected by one of skill in the art of organic synthesis. Solvents can be substantially reactive with the starting materials (reactants), the intermediates, or products under the conditions at which the reactions are carried out, i.e., temperature and pressure. Reactions can be carried out in one solvent or a mixture of more than one solvent. Product or intermediate formation can be monitored according to any suitable method known in the art. For example, product formulation can be monitored by spectroscopic means, such as nuclear magnetic resonance spectroscopy (e.g., ¹H or ¹³C), infrared spectroscopy, spectrophotometry (e.g., UV-visible), mass spectrometry, or by chromatography such as high-performance liquid chromatography (HPLC) or thin layer chromatography.

The disclosed compounds can be prepared by solid phase peptide synthesis wherein the amino acid α-N-terminal is protected by an acid or base protecting group. Such protecting groups should have the properties of being stable to the conditions of peptide linkage formation while being readily removable without destruction of the growing peptide chain or racemization of any of the chiral centers contained therein. Suitable protecting groups are 9-fluorenylmethyloxycarbonyl (Fmoc), t-butyloxycarbonyl (Boc), benzyloxycarbonyl (Cbz), biphenylisopropyloxycarbonyl, t-amyloxycarbonyl, isobornyloxycarbonyl, α,α-dimethyl-3,5-dimethoxybenzyloxycarbonyl, o-nitrophenylsulfenyl, 2-cyano-t-butyloxycarbonyl, and the like. The 9-fluorenylmethyloxycarbonyl (Fmoc) protecting group is particularly preferred for the synthesis of the disclosed compounds. Other preferred side chain protecting groups are: for side chain amino groups like lysine and arginine, 2,2,5,7,8-pentamethylchroman-6-sunfonyl (pmc), nitro, p-toluenesulfonyl, 4-methoxybenzene-sulfonyl, Cbz, Boc, and adamantyloxycarbonyl; for tyrosine, benzyl, o-bromobenzyloxycarbonyl, 2,6-dichlorobenzyl, isopropyl, t-butyl (t-Bu), cyclohexyl, cyclopentyl, and acetyl (Ac); for serine, t-butyl, benzyl, and tetrahydropyranyl; for histidine, trityl, benzyl, Cbz, p-toluenesulfonyl and 2,4-dinitrophenyl; for tryptophan, formyl; for aspartic and glutamic acid, benzyl and t-butyl; and for cysteine, triphenylmethyl (trityl). In solid phase peptide synthesis methods, the α-C-terminal amino acid is attached to a suitable solid support or resin. Suitable solid supports useful for the above synthesis are those materials that are inert to the reagents and reaction of the stepwise condensation-deprotection reactions, as well as being insoluble in the media use. Solid supports for synthesis of α-C-terminal carboxy peptides include 4-hydroxymethylphenoxymethyl-copoly(styrene-1% divinylbenzene) or 4-(2',4'-dimethoxyphenyl-Fmoc-aminomethyl)phenoxyacetamidoethyl resin available from Applied Biosystems (Foster City, CA). N,N'-diisopropylcarbodiimide (DIC) or O-benzotriazol-1-yl-N,N,N'N'-tetramethyluronium hexafluorophosphate (HBTU), with or without 4-dimethylaminopyridine (DMAP), 1-hydroxybenzotriazole (HOBt), benzotriazole-1-yloxy-tris(dimethylamino)phosphonium hexafluorophosphate (BOP) or bis(2-oxo-3-oxazolidinyl)phosphine chloride (BOPCl), mediate coupling for from about 0.5 to about 24 hours at a temperature of between 10 °C and 50 °C in a solvent such as dichloromethane, DMF, or NMP. When the solid support is 4-(2',4'-dimethoxyphenyl-Fmoc-aminomethyl)phenoxy-acetamidoethyl resin, the Fmoc group is cleaved with a secondary amine, preferably piperidine, prior to coupling with the α-C-terminal amino acid as described above. One method for coupling to the deprotected (3',4'-dimethoxyphenyl-Fmoc-aminomethyl)phenoxy-acetamidoethyl resin is O-benzotriazol-1-yl-N,N,N',N'-tetramethyluronium hexafluorophosphate (HBTU, 1 equiv.) and 1-hydroxybenzotriazole (HOBt, 1 equiv.) in DMF or O-(1H-6-chlorobenzotriazol-1-yl)-1,1,3,3,-tetramethyluronium hexafluorophosphate (HCTU, 1 equiv.) and N,N-diisopropylethylamine (DIEA, 1 equiv.) in NMP. The coupling of successive protected amino acids can be carried out in an automatic polypeptide synthesizer. In one example, the α-N-terminal in the amino acids of the growing peptide chain is protected with Fmoc. The removal of the Fmoc protecting group from the α-N-terminal side of the growing peptide is accomplished by treatment with a secondary amine, preferably piperidine. Each protected amino acid is then introduced in about 3-fold molar excess, and the coupling is preferably carried out in DMF. The coupling agent can be O-benzotriazol-1-yl-N,N,N',N'-tetramethyluronium hexafluorophosphate (HBTU, 1 equiv.) and 1-hydroxybenzotriazole (HOBt, 1 equiv.). At the end of the solid phase synthesis, the polypeptide is removed from the resin and deprotected, either successively or in a single operation. Removal of the polypeptide and deprotection can be accomplished in a single operation by treating the resin-bound polypeptide with a cleaving reagent comprising thianisole, water, ethanedithiol, and trifluoroacetic acid. In cases wherein the α-C-terminal of the polypeptide is an alkylamide, the resin is cleaved by aminolysis with an alkylamine. Alternatively, the peptide can be removed by transesterification, e.g., with methanol, followed by aminolysis or by direct transamidation. The protected peptide can be purified at this point or taken to the next step directly. The removal of the side chain protecting groups can be accomplished using the cleavage cocktail described above. The fully deprotected peptide can be purified by a sequence of chromatographic steps employing any or all of the following types: ion exchange on a weakly basic resin (acetate form); hydrophobic adsorption chromatography or underivatized polystyrene-divinylbenzene (for example, Amberlite XED); silica gel adsorption chromatography; ion exchange chromatography on carboxymethylcellulose; partition chromatography, e.g., on Sephadex G-25, LH-20 or countercurrent distribution; or high performance liquid chromatography (HPLC), especially reverse-phase HPLC on octyl- or octadecylsilyl-silica bonded column packing.

### Pharmaceutical Compositions

Also disclosed are pharmaceutical compositions comprising any of the polypeptides disclosed herein in a pharmaceutically acceptable carrier. The disclosed polypeptides can be incorporated in the formulations described below as neutral compounds, pharmaceutically acceptable salts, and/or prodrugs thereof. Pharmaceutical formulations can be designed for immediate release, sustained release, delayed release, and/or a burst release of one or more of the disclosed polypeptides in a therapeutically effective amount.

The compounds described herein can be formulated for parenteral administration. Parenteral formulations can be prepared as aqueous compositions using techniques known in the art. Typically, such compositions can be prepared as injectable formulations, for example, solutions or suspensions; solid forms suitable for use in preparing solutions or suspensions upon the addition of a reconstitution medium prior to injection; emulsions, such as water-in-oil (w/o) emulsions, oil-in-water (o/w) emulsions, and microemulsions thereof; liposomes, or emulsomes.

The carrier can be a solvent or dispersion medium containing, for example, water, ethanol, one or more polyols (e.g., glycerol, propylene glycol, and liquid polyethylene glycol), oils such as vegetable oils (e.g., peanut oil, corn oil, sesame oil, etc.) and combinations thereof.

Solutions and dispersions of the polypeptides as neutral compounds or pharmaceutically acceptable salts thereof can be prepared in water or another solvent or dispersion medium suitable mixed with one or more pharmaceutically acceptable excipients including, but not limited to, surfactants, dispersants, emulsifiers, pH modifying agents, or combinations thereof.

Suitable surfactants for use in the disclosed pharmaceutical compositions may be anionic, cationic, amphoteric, or nonionic surface active agents. Suitable anionic surfactants include but are not limited, to, carboxylate, sulfonate, and sulfate ions. Examples of anionic surfactants include sodium, potassium, and/or ammonium salts of long chain alkyl sulfonates and alkyl aryl sulfonates such as sodium dodecylbenzene sulfonate; dialkyl sodium sulfosuccinates, such as sodium dodecylbenzene sulfonate and sodium bis-(2-ethylthioxyl)-sulfosuccinate; and alkyl sulfates such as sodium lauryl sulfate. Cationic surfactants include but are not limited to, quaternary ammonium compounds such as benzalkonium chloride, benzethonium chloride, cetrimonium bromide, stearyl dimethylbenzyl ammonium chloride, polyoxyethylene, and coconut amine. Examples of nonionic surfactants include ethylene glycol monostearate, propylene glycol myristate, glyceryl monostereate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer 401, stearoyl monoisopropanolamide, and polyoxyethylene hydrogenated tallow amide. Examples of amphoteric surfactants include sodium N-dodecyl-β-alanine, sodium-N-lauryl-β-iminodipropionate, myristoamphoacetate, lauryl betaine, and lauryl sulfobetaine.

The formulation can contain a preservative to prevent the growth of microorganisms. Suitable preservatives include but are not limited to, parabens, chlorobutanol, phenol, sorbic acid, and thimerosal. The formulation may also contain an antioxidant to prevent degradation of the one or more polypeptides.

The formulation is typically buffered to a pH of 3 -8 for parenteral administration upon reconstitution. Suitable buffers include but are not limited to, phosphate buffers, acetate buffers, and citrate buffers.

Water soluble polymers are often used in formulations for parenteral administration. Suitable water-soluble polymers include but are not limited to, polyvinylpyrrolidone, dextran, carboxymethylcellulose, and polyethylene glycol.

Sterile injectable solutions can be prepared by incorporating the one or more polypeptides described herein in the required amount in the appropriate solvent or dispersion medium with one or more of the excipients listed above, as required, followed by filtered sterilization. Generally, dispersions are prepared by incorporating the various sterilized active ingredients into a sterile vehicle which contains the basic dispersion medium and the required additional ingredients from those listed above. In the case of sterile powders for the preparation of sterile injectable solutions, the preferred methods of preparation are vacuum-drying and freeze-drying techniques, which yield a powder of the one or more disclosed polypeptides plus any additional desired ingredient from a previously sterile-filtered solution thereof. The powders can be prepared in such a manner that the particles are porous in nature, which can increase dissolution of the particles. Methods for making porous particles are well-known in the art.

The parenteral formulations described herein can be formulated for controlled release, including immediate release, delayed release, extended release, pulsatile release, and combinations thereof. For parenteral administration, the polypeptides described herein and, optionally, one or more additional active agents can be incorporated into microparticles, nanoparticles, or combinations thereof that provide controlled release. For example, the compounds and/or one or more additional active agents can be incorporated into polymeric microparticles, which provide controlled release of the polypeptide(s). Release of the polypeptide(s) is controlled by diffusion of the polypeptide(s) out of the microparticles and/or degradation of polymeric particles by hydrolysis and/or enzymatic degradation. Suitable polymers include ethylcellulose and other natural or synthetic cellulose derivatives.

Polymers which are slowly soluble and form a gel in an aqueous environment, such as hydroxypropyl methylcellulose or polyethylene oxide may also be suitable as material for drug-containing microparticles. Other polymers include, but are not limited to, polyanhydrides, poly(ester anhydrides), polyhydroxyacids such as polylactide (PLA), polyglycolide (PGA), poly(lactide-co-glycolide) (PLGA), poly-3-hydroxybutyrate (PHB) and copolymers thereof, poly-4-hydroxybutyrate (P4HB) and copolymers thereof, polycaprolactone and copolymers thereof, and combinations thereof.

The polypeptide can also be formulated for depot injection. In a depot injection, the active agent is formulated with one or more pharmaceutically acceptable carriers that provide for the gradual release of the polypeptide over a period of hours or days after injection. The depot formulation can be administered by any suitable means; however, the depot formulation is typically administered via subcutaneous or intramuscular injection. A variety of carriers may be incorporated into the depot to provide for the controlled release of the active agent. In some cases, depot formulations contain one or more biodegradable polymeric or oligomeric carriers. Suitable polymeric carriers include but are not limited to, poly(lactic acid) (PLA), poly(lactic-co-glycolic acid) (PLGA), poly(lactic acid)-polyethylene glycol (PLA-PEG) block copolymers, polyanhydrides, poly(ester anhydrides), poly(glycolic acid) (PGA), poly-3-hydroxybutyrate (PHB) and copolymers thereof, poly-4-hydroxybutyrate (P4HB) and copolymers thereof, polycaprolactone, cellulose, hydroxypropyl methylcellulose, ethylcellulose, as well as blends, derivatives, copolymers, and combinations thereof. In depot formulations containing a polymeric or oligomeric carrier, the carrier, and the polypeptide can be formulated as a solution, an emulsion, or a suspension. One or more polypeptides, and optionally one or more additional active agents, can also be incorporated into polymeric or oligomeric microparticles, nanoparticles, or combinations thereof.

Formulations may also be in the form of an organogel (assuming the polypeptide is relatively water-insoluble) or a hydrogel. Numerous gel formulations are known. See, for example, U.S. Patent No. 5,411,737. Hydrogels, especially those further including nanoparticles or microparticles for sustained, immediate, and/or delayed release, can also be used.

Oral pharmaceutical dosage forms are either solid, gel, or liquid. The solid dosage forms are tablets, capsules, granules, and bulk powders. Types of oral tablets include compressed, chewable lozenges and tablets, which may be enteric-coated, sugar-coated, or film-coated. Capsules may be hard or soft gelatin capsules, while granules and powders may be provided in non-effervescent or effervescent form with the combination of other ingredients known to those skilled in the art.

The polypeptides may be formulated for local or topical application, such as for topical application to the skin and mucous membranes, such as in the eye, in the form of gels, creams, and lotions, and for application to the eye or for intracisternal or intraspinal application. Topical administration is contemplated for transdermal delivery and also for administration to the eyes or mucosa or for inhalation therapies. Nasal solutions of the polypeptides alone or in combination with other pharmaceutically acceptable excipients can also be administered. These solutions, particularly those intended for ophthalmic use, may be formulated as 0.01% - 10% isotonic solutions, having a pH of about 5-7, with appropriate salts.

Other routes of administration, such as transdermal patches, including iontophoretic and electrophoretic devices, and vaginal and rectal administration, are also contemplated herein. Transdermal patches, including iontophoretic and electrophoretic devices, are well known to those of skill in the art. For example, pharmaceutical dosage forms for rectal administration are rectal suppositories, capsules, and tablets for systemic effect. Rectal suppositories, as used herein, mean solid bodies for insertion into the rectum, which melt and soften at body temperature, releasing one or more pharmacologically or therapeutically active ingredients. Pharmaceutically acceptable substances utilized in rectal suppositories are bases or vehicles and agents to raise the melting point. Examples of bases include cocoa butter (Theobroma oil), glycerin-gelatin, carbowax (polyoxyethylene glycol), and appropriate mixtures of mono-, di-, and triglycerides of fatty acids. Combinations of the various bases may be used. Agents to raise the melting point of suppositories include spermaceti and wax. Rectal suppositories may be prepared either by the compressed method or by molding. The weight of a rectal suppository, in one aspect, is about 2 to 3 g.

### Methods of Treatment

The polypeptides described herein can be used to treat or prevent a disease, disorder, or condition in a patient in need thereof. In some aspects, treatment refers to partial or complete alleviation, amelioration, relief, inhibition, delaying onset, reducing severity, and/or incidence of the disease, disorder, or condition in the patient.

The terms "improve," "increase," "reduce," "decrease," and the like, as used herein, indicate values that are relative to a control. In some aspects, a suitable control is a baseline measurement, such as a measurement in the same individual prior to initiation of the treatment described herein or a measurement in a control individual (or multiple control individuals) in the absence of the treatment described herein.

In some aspects, the patient is an individual who has recently been diagnosed with a disease, disorder, or condition. Typically, early treatment (treatment commencing as soon as possible after diagnosis) is important to minimize the effects of the disease, disorder, or condition and to maximize the benefits of treatment.

In some aspects, the polypeptides described herein can be used to treat or prevent a neurological disease or condition. In some aspects, the disease, disorder, or condition is a neurodegenerative disease. In some aspects, the neurological or neurogenerative disease or condition that may be treated includes, for example, Alzheimer's disease, cerebral edema, cerebral ischemia, multiple sclerosis, neuropathies, Parkinson's disease, Huntington's disease, blunt or surgical trauma (including postsurgical cognitive dysfunction and spinal cord or brain stem injury), as well as neurological aspects of disorders such as degenerative disc disease and sciatica.

Further examples of neurodegenerative disorders include but are not limited to, Alexander's disease, Alper's disease, Alzheimer's disease, amyotrophic lateral sclerosis, ataxia telangiectasia, Batten disease, bovine spongiform encephalopathy, Canavan disease, Cockayne syndrome, corticobasal degeneration, Creutzfeldt-Jakob disease, Huntington's disease, HIV-associated dementia, Kennedy's disease, Krabbe's disease, Lewy body dementia, Machado-Joseph disease, multiple sclerosis, multiple system atrophy, narcolepsy, neuroborreliosis, Parkinson's disease, Pelizaeus-Merzbacher disease, Pick's disease, primary lateral sclerosis, prion diseases, Refsum's disease, Sandhoff's disease, Schilder's disease, subacute combined degeneration of spinal cord secondary to pernicious anemia, schizophrenia, spinocerebellar ataxia, spinal muscular atrophy, Steele-Richardson-Olszewski disease, and tabes dorsalis.

In some aspects, a neurodegenerative disease includes any pathological state involving neuronal degeneration, including Parkinson's disease, Huntington's disease, Alzheimer's disease, and amyotrophic lateral sclerosis. Polyglutamine diseases, including Huntington's disease, are neurodegenerative diseases caused by an abnormally expanded polyglutamine tract in the causative gene products.

Thus in one aspect, a method of treating a neurodegenerative disease in a subject is provided, comprising administering to the subject a therapeutically effective amount of a polypeptide described herein. In some aspects, the subject is an individual suffering from or susceptible to a neurodegenerative disease. In some aspects, the subject is a human.

In some aspects, a method of treating Parkinson's disease in a subject is provided comprising administering to the subject a therapeutically effective amount of a polypeptide described herein.

In some aspects, a method of treating Huntington's disease in a subject is provided comprising administering to the subject a therapeutically effective amount of a polypeptide described herein.

In some aspects, a method of treating Alzheimer's disease in a subject is provided comprising administering to the subject a therapeutically effective amount of a polypeptide described herein.

In some aspects, a method of treating amyotrophic lateral sclerosis (ALS) in a subject is provided, comprising administering to the subject a therapeutically effective amount of a polypeptide described herein.

In another aspect, a method is provided for treating a disorder or condition that is treated by inhibiting or decreasing protein-protein interactions between LRRK2 and FADD in a subject in need thereof, the method comprising administering to the subject a therapeutically effective amount of a polypeptide described herein.

In another aspect, a method is provided for treating or preventing nerve cell degeneration, the method comprising administering to a subject suffering or susceptible to nerve cell degeneration a therapeutically effective amount of a polypeptide described herein.

In particular aspects, the disorder or condition comprises Parkinson's disease or a Parkinson-plus syndrome. Parkinson-plus syndromes include multiple system atrophy and progressive supranuclear party (PSP). In certain aspects, the polypeptides described herein are used to treat Parkinson's disease that presents in one or more forms, including, but not limited to, sporadic Parkinson's disease, a familial form of Parkinson's disease, autosomal recessive early-onset Parkinson's disease, or post-encephalitic Parkinson's disease. In some aspects, a therapeutically effective amount of the polypeptides described herein, when administered to a subject having Parkinson's disease or a Parkinson-plus syndrome, ameliorates or lessens the severity of one or more of the symptoms of the disease, including but not limited to tremor, rigidity of the limbs and trunk, akinesia, bradykinesia, and postural abnormalities.

The polypeptides provided herein can treat the described diseases, disorders, or conditions, for instance, by disrupting protein-protein interactions between LRRK2 and FADD.

In some aspects, the polypeptides described herein may be used in a method of treating a disorder or condition selected from: Parkinson's disease; migraine; epilepsy; Alzheimer's disease; brain injury; stroke; cerebrovascular disease (including cerebral arteriosclerosis, cerebral amyloid angiopathy, hereditary cerebral hemorrhage, and brain hypoxia-ischemia); cognitive disorders (including amnesia, senile dementia, HIV-associated dementia, Alzheimer's disease, Huntington's disease, Lewy body dementia, vascular dementia, drug-related dementia, tardive dyskinesia, myoclonus, dystonia, delirium, Pick's disease, Creutzfeldt-Jacob disease, HIV disease, Gilles de la Tourette's syndrome, epilepsy, muscular spasms and disorders associated with muscular spasticity or weakness including tremors, and mild cognitive impairment); mental deficiency (including spasticity, Down syndrome and fragile X syndrome); sleep disorders (including hypersomnia, circadian rhythm sleep disorder, insomnia, parasomnia, and sleep deprivation); psychiatric disorders such as anxiety (including acute stress disorder, generalized anxiety disorder, social anxiety disorder, panic disorder, post-traumatic stress disorder, agoraphobia, and obsessive compulsive disorder); factitious disorder (including acute hallucinatory mania); impulse control disorders (including compulsive gambling and intermittent explosive disorder); mood disorders (including bipolar I disorder, bipolar II disorder, mania, mixed affective state, major depression, chronic depression, seasonal depression, psychotic depression, premenstrual syndrome (PMS), premenstrual dysphoric disorder (PDD), and postpartum depression); psychomotor disorder; psychotic disorders (including schizophrenia, schizoaffective disorder, schizophreniform, and delusional disorder); drug dependence (including narcotic dependence, alcoholism, amphetamine dependence, cocaine addiction, nicotine dependence, and drug withdrawal syndrome); eating disorders (including anorexia, bulimia, binge eating disorder, hyperphagia, obesity, compulsive eating disorders and pagophagia); sexual dysfunction disorders; urinary incontinence; neuronal damage disorders (including ocular damage, retinopathy or macular degeneration of the eye, tinnitus, hearing impairment and loss, and brain edema) and pediatric psychiatric disorders (including attention deficit disorder, attention deficit/hyperactivity disorder, conduct disorder, and autism) in a subject, preferably a human, wherein the method comprises administering to a subject a therapeutically effective amount of the polypeptides described herein.

Other disorders which may be treated with the compounds described herein include but are not limited to, lysosomal disorders (for example, Niemann-Pick Type C disease, Gaucher disease), Crohn's disease, thyroid, renal (including papillary renal), breast, lung, and prostate cancers, leukemias (including acute myelogenous leukemia), lymphomas, multiple sclerosis, rheumatoid arthritis, system lupus erythematosus, autoimmune hemolytic anemia, pure red cell aplasia, idiopathic thrombocytopenic purpura (ITP), Evans syndrome, vasculitis, bullous skin disorders, type 1 diabetes mellitus, Sjogren's syndrome, Devic's disease, and inflammatory myopathies.

In some aspects, a method of treating Crohn's disease in a subject in need thereof is provided comprising administering a therapeutically effective amount of a synthetic peptide described herein to the subject.

A number of embodiments of the disclosure have been described. Nevertheless, it will be understood that various modifications may be made without departing from the spirit and scope of the invention. Accordingly, other embodiments are within the scope of the following claims.

By way of non-limiting illustration, examples of certain embodiments of the present disclosure are given below.

### EXAMPLES

The following examples are put forth to provide those of ordinary skill in the art with a complete disclosure and description of how the compounds, compositions, and methods claimed herein are made and evaluated and are intended to be purely exemplary of the invention and are not intended to limit the scope of what the inventors regard as their invention. Efforts have been made to ensure accuracy concerning numbers (e.g., amounts, temperature, etc.), but some errors and deviations should be accounted for. Unless indicated otherwise, parts are parts by weight, temperature is in degrees Celsius or is at ambient temperature, and pressure is at or near atmospheric pressure.

### Example 1. Constrained peptides disrupt the LRRK2-FADD interaction and are neuroprotective

This example aimed to determine if peptide-based compounds could provide neuroprotection by disrupting the interaction between mutant LRRK2 and FADD. Constrained peptides were designed mimicking the alpha-helical protein interaction interface between the LRRK2 armadillo region and the death domain of FADD. These peptide-based protein-protein interaction inhibitors significantly reduced this interaction and blocked apoptotic death of primary neurons expressing G2019S-LRRK2 without affecting the kinase activity of LRRK2. This example has identified novel constrained peptides that disrupt mutant LRRK2-induced neuronal death in an allosteric manner, thereby providing a potential alternative therapeutic approach for Parkinson's disease.

In this example, the goal was to extend previous findings by developing an alternative approach to de-stabilize the LRRK2-FADD PPI using constrained ("stapled") peptides. This approach to disrupting this PPI was to design a series of hydrocarbon-stapled peptides based on the sequence within FADD that binds to LRRK2. We utilized the crystal structure of the FADD death domain and the model we previously constructed of the LRRK2-ARM region in order to identify key residues that comprise the PPI interface. A library of hydrocarbon-constrained peptides was characterized for their ability to inhibit LRRK2-FADD interactions.

Herein, a constrained peptide mimicking a portion of the modeled FADD-LRRK2 interface, termed FARMS, disrupts the interaction between LRRK2 and FADD and results in neuroprotection in a cellular model of mutant LRRK2-PD. The peptide permeates cells using the HEK293T cell line as well as primary cortical neurons. Further, the PPI between LRRK2 and FADD is disrupted by the FARMS peptide. Critically, blocking the interaction between mutant LRRK2 and FADD in primary neurons by FARMS prevents the initiation of a FADD-dependent cell death pathway and subsequent apoptotic neuronal death. Overall, these findings demonstrate a novel, non-catalytic strategy to inhibit LRRK2 from inducing neuronal apoptosis in Parkinson's disease.

### Results

*Design and synthesis of constrained peptides targeting LRRK2 PPIs.* Using the modeled binding interface between the LRRK2 ARM region and the death-domain (DD) of FADD as a template, key residues were identified that comprised the binding interface with LRRK2 (FIGs. 1A and 1B). This FADD-derived helix (residues (173-190) were used for the library design of FADD Armadillo domain (FARM) peptides (FIG. 1B). The FARM peptide library was synthesized using standard solid phase peptide synthesis. The staple was generated by incorporating (S)-2-(4-pentenyl)alanine at i, i+4 positions and cyclized using ring-closing metathesis (RCM) chemistry while on solid support (FIG. 1C). The library (FARM1-FARM5) was generated by integrating the staple at different positions along the predicted non-binding side of the helix to stabilize side chain residues that were anticipated to bind to LRRK2 (FIG. 1D). In addition, Lys residues were added to the terminal ends of each peptide to increase the overall positive net charge to promote cell permeation while also improving water solubility.

*Constrained peptides disrupt the PPI between LRRK2 and FADD.* It was previously shown that deletion of a region within the LRRK2 armadillo region disrupts the interaction with FADD and is neuroprotective. Herein, an alternative approach was taken to disrupting this PPI by the competitive binding of a conformationally constrained peptide based on the LRRK2 binding region in the FADD death domain. First, it was assessed whether the constrained peptides could be taken up by cells in culture via addition to the growth medium. HEK293T cells were treated with FAM-labeled FARM peptide (in this representative image, FARMS) or its native control (a peptide with the identical sequence, but without the staple) for 24 hr, fixed and stained with a nuclear dye. While the non-stapled control peptide was not found to permeate cells, a clear diffuse fluorescent signal along with a few visible puncta was observed throughout cells treated with FARM5 (FIG. 2A). Additionally, the uptake of FAM-labeled peptide is dose-dependent over a 24hr period (FIG. 2B). This confirms previous findings (see Helton LG, Soliman A, von Zweydorf F, Kentros M, Manschwetus JT, Hall S, Gilsbach B, Ho FY, Athanasopoulos PS, Singh RK, LeClair TJ, Versees W, Raimondi F, Herberg FW, Gloeckner CJ, Rideout H, Kortholt A, Kennedy EJ (2021) Allosteric Inhibition of Parkinson's-Linked LRRK2 by Constrained Peptides. ACS Chem Biol 16, 2326-2338) indicating that the presence of the peptide staple markedly facilitates cellular uptake.

It was then assessed whether this peptide library (FARM1-FARM5) could disrupt the interaction between over-expressed Flag G2019S-LRRK2 and V5-FADD by co-immunoprecipitation. Flag-LRRK2 was purified on anti-Flag resin and the eluate as well as the protein extract (input) separated by SDS-PAGE, with the membranes probed for V5 (FADD) and Flag (LRRK2). The transfected cells were treated with the indicated peptide (10 µM) for a total of 48 hr prior to collection. At this concentration and time point, a significant reduction was seen in the amount of FADD that co-purified with G2019S-LRRK2 (FIGs. 3A and 3D), when normalized to the amount of immunoprecipitated Flag-LRRK2. The dose-dependency of the disruption of this interaction by FARMS over a 24hr period was further assessed, and found a progressive reduction in the LRRK2-FADD PPI with increasing doses from 0.1 to 10 µM was found (see FIGs. 3B and 3D). The greatest, and most consistent, reduction appeared to be with the FARMS peptide (see FIGs. 3C and 3D); however the other peptides, which have the same sequence but differ in the placement of the hydrocarbon "staple", also reduced this interaction, but to a lesser extent. To minimize the use of primary mouse cortical neuron cultures, FARMS was used in subsequent experiments to examine the potential neuroprotective effects of LRRK2-FADD disruption.

*Disruption of the LRRK2-FADD PPI is neuroprotective.* It has been previously shown that over-expression of mutant LRRK2 (G2019S or other pathogenic mutations) in primary cortical neurons results in apoptotic death of the neurons (see Iaccarino C, Crosio C, Vitale C, Sanna G, Carri MT, Barone P (2007) Apoptotic mechanisms in mutant LRRK2-mediated cell death. Hum Mol Genet 16, 1319-1326); and this example continues this area of identifying pathway(s) underlying cell-autonomous neuronal death in models of LRRK2-PD. Prior studies characterizing the pathologic interaction between LRRK2 and FADD have demonstrated the importance of this PPI in transducing cell-autonomous neuronal death induced by mutant LRRK2. Disruption of this interaction by dominant negative fragments of both FADD and LRRK2, or by deletion of the predicted FADD-binding domain within LRRK2, is neuroprotective against over-expression of mutant LRRK2. To determine whether FARMS could effectively disrupt the apoptotic death of primary neurons induced by pathogenic LRRK2, mouse embryonic cortical neurons were transiently transfected with G2019S-LRRK2 and increasing concentrations of the FARMS peptide were added. In cultures of neurons expressing G2019S-LRRK2, apoptotic Flag-positive neurons with characteristic fragmented and condensed nuclear apoptotic features, were observed as has been reported previously. In contrast, neurons expressing G2019S-LRRK2 treated with 1 µM FARMS showed reduced evidence of chromatin condensation (i.e., apoptotic neuronal death; see FIG. 4A). Fluorescent signal from the FAM peptide tag was observed diffusely distributed throughout most neurons (FIG. 4A). A third neuron is visible within this field (with a condensed nucleus), however it is not expressing Flag-LRRK2, so would not be included in the assessment of neuronal death. the dose response of neuroprotection of FARMS was determined by counting the percentage of transfected neurons (stained with anti-Flag antibodies). The lowest concentration, 0.1 µM of FARMS, was unable to significantly block apoptotic neuronal death. However, at higher concentrations, 1 and 10 µM, the percentage of neurons with apoptotic profiles was significantly reduced compared to vehicle treated neurons (FIG. 4B). This indicates that disrupting the LRRK2-FADD with a FADD-derived constrained peptide can effectively protect primary neurons from the cell-autonomous death pathway triggered by over-expression of mutant LRRK2. It should be noted again that these data rely on an over-expression model in primary neurons; which to this date is the only acute cellular model in which neuronal death is observed. Alternative cell models (e.g. iPS-differentiated dopaminergic neurons) do not exhibit neuronal death at endogenous levels.

*Loss of the LRRK2-FADD PPI does not affect LRRK2 kinase activity.* Since the peptides were designed to block LRRK2 from interacting with FADD, they should not have any effect on the catalytic kinase activity of LRRK2. To determine whether the PPI-disrupting FARMS peptide alters LRRK2 kinase activity in cells, HEK293T cells were co-transfected with WT or G019S-LRRK2 together with Rab10. Cells over-expressing mutant LRRK2 and human WT RablO were treated with FARMS and the cell extracts were probed for multiple indices of LRRK2 kinase activity including phosphorylated Rab10 and pS1292-LRRK2. As a positive control for inhibition of LRRK2 activity, parallel cells were treated with the potent catalytic inhibitor of LRRK2, MLi-2 (see Fell MJ, Mirescu C, Basu K, Cheewatrakoolpong B, DeMong DE, Ellis JM, Hyde LA, Lin Y, Markgraf CG, Mei H, Miller M, Poulet FM, Scott JD, Smith MD, Yin Z, Zhou X, Parker EM, Kennedy ME, Morrow JA (2015) MLi-2, a Potent, Selective, and Centrally Active Compound for Exploring the Therapeutic Potential and Safety of LRRK2 Kinase Inhibition. J Pharmacol Exp Ther 355, 397-409.).

In the representative western immunoblot depicted in FIG. 5A, levels of total and phosphorylated Rab10 (T73) and LRRK2 (S1292) are shown. Cells co-expressing G2019S-LRRK2 and RablO exhibit a robust increase in pRab10 levels as compared to WT-LRRK2 expressing cells or cells treated with MLi-2 (FIG. 5A), reflective of the expected increase in kinase activity. Additionally, levels of pS1292-LRRK2 were increased as expected in cells expressing G2019S-LRRK2, which was prevented by treatment with MLi-2 (FIG. 5A). In contrast to the inhibition of auto-phosphorylation and phosphorylation of RablO by MLi-2, any loss in phosphorylation of pS1292-LRRK2 or RablO could be detected (pT73; FIG. 5A). A qualitative summary of the effects of FARMS on LRRK2 kinase activity in cells is shown in FIG. 5B. Surprisingly, there was an unexpected increase in the phosphorylation of RablO in G2019S-expressing cells treated with FARMS that does not appear to be dose-dependent. There are several potential reasons for this response (see Discussion below). At endogenous levels, RablO showed the expected increase in pT73 levels in cells over-expressing R1441C-LRRK2 (FIG. 5C); but this was un-affected by treatment with increasing doses of FARMS. However, while it is unclear why phosphorylation of RablO was enhanced in a cellular setting, it demonstrates that these peptides inhibit the interaction between LRRK2 and FADD while leaving its kinase catalytic activity intact.

### Discussion

In this example, the critical PPI between LRRK2 and the cell death adaptor protein FADD underlying the apoptotic death of neurons exogenously expressing pathogenic mutant forms of LRRK2 was characterized. This example shows that constrained peptides mimicking a helix derived from the FADD death domain that binds to the LRRK2 ARM domain can effectively disrupt this PPI, thereby inhibiting apoptotic death of primary neurons over-expressing the pathogenic mutant form of LRRK2 (G2019S). Given the evidence that LRRK2 may also play a vital role in the more common idiopathic forms of the disease (iPD) where wild-type LRRK2 is present (see Di Maio R, Hoffman EK, Rocha EM, Keeney MT, Sanders LH, De Miranda BR, Zharikov A, Van Laar A, Stepan AF, Lanz TA, Kofler JK, Burton EA, Alessi DR, Hastings TG, Greenamyre JT (2018) LRRK2 activation in idiopathic Parkinson's disease. Sci Transl Med 10**;** and Melachroinou K, Kang MS, Liong C, Narayan S, Levers N, Joshi N, Kopil K, Hutten SJ, Baptista MAS, Padmanabhan S, Kang UJ, Stefanis L, Alcalay RN, Rideout HJ (2020) Elevated In Vitro Kinase Activity in Peripheral Blood Mononuclear Cells of Leucine-Rich Repeat Kinase 2 G2019S Carriers: A Novel Enzyme-Linked Immunosorbent Assay-Based Method. Mov Disord 35, 2095-2100), it will be critical to determine if this PPI-targeting approach can also demonstrate protection in cellular and *in vivo* models of iPD. At this point, it remains unknown whether the LRRK2-FADD PPI is also required for neuronal loss in iPD; and conversely, it is also not known what key phospho-substrate(s) of LRRK2 are required for the death of dopaminergic neurons. Moreover, since this example addresses only the cell-autonomous neuronal death signaling triggered by mutant LRRK2, any potential therapeutic approach targeting this PPI may also need to consider non cell-autonomous mechanisms that contribute to the ultimate loss of DA neurons in PD; possible requiring additional LRRK2 protein interactors.

Initial characterization of the LRRK2-FADD pathway indicated that it is triggered downstream of kinase activity. Genetic inhibition of LRRK2 kinase activity (K1906R mutation) only restores the interaction to wildtype levels but does not eliminate it, suggesting a broader potential role for this PPI in PD. This example shows that the interaction of LRRK2 with FADD is not required for kinase activity. Displacement of FADD from the LRRK2 N-terminal region did not lead to a broad inhibition of kinase activity; in fact, FADD binding to LRRK2 may diminish access to some substrates. We found that treatment of cells with the FARMS peptide led to increased phosphorylation of over-expressed Rab10. The fact that LRRK2 kinase activity remained, despite having the interaction with FADD disrupted by the targeted stapled peptides, suggests that LRRK2 dimerization is also unaffected given that this is a crucial step in activating its kinase function. This suggests that there is a kinase-independent regulation of this interaction as well, similar to the scaffolding function performed by LRRK2 in RIPK1-dependent apoptosis (see Amin P, Florez M, Najafov A, Pan H, Geng J, Ofengeim D, Dziedzic SA, Wang H, Barrett VJ, Ito Y, LaVoie MJ, Yuan J (2018) Regulation of a distinct activated RIPK1 intermediate bridging complex I and complex II in TNFalpha-mediated apoptosis. Proc Natl Acad Sci USA 115, E5944-E5953).

In attempting to rule out an impact on LRRK2 kinase activity as a potential confounding mechanism for the neuroprotection provided by the FARMS peptide in primary neurons, an unexpected consequence of disruption of FADD binding was uncovered. In HEK293T cells co-expressing G2019S-LRRK2 and human Rab10, treatment with the FARMS peptide resulted in an *increase* in LRRK2-dependent phosphorylation of RablO (Thr73). In contrast, auto-phosphorylation of LRRK2 at Ser1292 was un-affected by treatment with the FARMS peptide, suggesting that kinase activity is not reduced when the LRRK2-FADD PPI is disrupted. One possible explanation might be that FADD binding within the ARM repeat region of the LRRK2 N-terminus may interfere with docking of RablO, particularly since it was previously shown that the interaction of FADD at this location requires its dimerization. Mutant F25R FADD, which blocks its dimerization, displays markedly reduced binding to LRRK2. A recent report identifying two N-terminal RablO binding sites in LRRK2 is consistent with this hypothesis (see Vides EG, Adhikari A, Chiang CY, Lis P, Purlyte E, Limouse C, Shumate JL, Spinola-Lasso E, Dhekne HS, Alessi DR, Pfeffer SR (2022) A feed-forward pathway drives LRRK2 kinase membrane recruitment and activation. Elife 11**).** The first binding motifs, binding preferentially *non-*phosphorylated Rab GTPases (Rab8a, Rab10, Rab29), are located within the 350-500 amino acid region of the LRRK2 ARM, precisely in the region where binding of FADD was previously mapped, between amino acids 500 and 550. This is supported by the observation that pS1292-LRRK2 levels are unchanged with exposure to FARMS, indicating that the intrinsic kinase activity of G2019S-LRRK2 is not affected by displacement of FADD from the ARM region of LRRK2, which then provides greater access to the Rab10 substrate. By extension, since pS1292 levels are equally elevated in cells expressing G2019S-LRRK2 plus treatment with FARMS, it could be predicted that LRRK2 dimerization is also unaffected by displacement of FADD by the FARMS peptide. Of note, and it remains to be reconciled, is the aggregate observations of increased binding of R1441C-LRRK2 to FADD, and robustly increased phosphorylation of Rab10 by this particular LRRK2 variant (for example, see Iannotta L, Biosa A, Kluss JH, Tombesi G, Kaganovich A, Cogo S, Plotegher N, Civiero L, Lobbestael E, Baekelandt V, Cookson MR, Greggio E (2020) Divergent Effects of G2019S and R1441C LRRK2 Mutations on LRRK2 and Rab10 Phosphorylations in Mouse Tissues. Cells 9). This also raises the possibility that elevated phosphorylation of RablO specifically may not be critical for the cell-autonomous neuronal death elicited by mutant LRRK2. It should be noted that the apparent increase in Rab10 phosphorylation was only evident in an over-expression setting, and not with endogenous levels of Rab10 (FIGs. 5A-5C). A more thorough investigation of this apparent competitive binding of FADD and Rab10 to this region within LRRK2 is being undertaken.

Mutant LRRK2-induced PD likely involves multiple cell and non-cell autonomous mechanisms in the pathogenesis of the disease, given its expression and known roles in both neuronal and non-neuronal cells (see Oun A, Sabogal-Guaqueta AM, Galuh S, Alexander A, Kortholt A, Dolga AM (2022) The multifaceted role of LRRK2 in Parkinson's disease: From human iPSC to organoids. Neurobiol Dis 173, 105837). The enriched primary neuronal model that employed, like others have also reported, elicits a cell autonomous neuronal death; which is not to say that non-cell autonomous mechanisms are not equally vital to the disease pathogenesis or progression. It is also critical to note that this acute model of neuronal death induced by mutant LRRK2 (G2019S or other pathogenic mutants), requires the over-expression of the transgene in primary neurons. Neuronal death is generally not observed at endogenous expression levels in neurons, at least during the acute time periods employed in the literature. Disruption of this PPI is effective not only against neuronal death elicited by G2019S-LRRK2 expression, but also other pathogenic LRRK2 variants (see Melachroinou K, Leandrou E, Valkimadi PE, Memou A, Hadjigeorgiou G, Stefanis L, Rideout HJ (2016) Activation of FADD-Dependent Neuronal Death Pathways as a Predictor of Pathogenicity for LRRK2 Mutations. PLoS One 11, e0166053). The highest concentration of FARMS restored neuronal death to WT levels (see FIGs. 4A-4B) within the isolated, cell-autonomous, cellular model. It will be critical to also determine if this PPI regulates or underlies LRRK2-dependent degeneration mediated by signaling in other non-neuronal cell types.

Several advantages of using constrained peptides are their ability to bind large protein surfaces such as that in PPIs due to the large surface area presented by the peptide, but also their high affinity, specificity, and low immunogenicity (see Morrison C (2018) Constrained peptides' time to shine? Nat Rev Drug Discov 17, 531-533). While these peptides were shown to permeate cells and neurons, a major hurdle still exists in delivering peptides across the blood-brain barrier (BBB). Utilization of delivery methods such as intranasal or intracranial delivery may overcome some of these challenges, but other strategies such as carrier-mediated transporters or receptor-mediated transporters may also be employed for brain delivery (see Pardridge WM (2019) Blood-Brain Barrier and Delivery of Protein and Gene Therapeutics to Brain. Front Aging Neurosci 11, 373). Nonetheless, LRRK2 also plays an important role in peripheral tissues and immune cells in PD (see Wallings RL, Tansey MG (2019) LRRK2 regulation of immune-pathways and inflammatory disease. Biochem Soc Trans 47, 1581-1595). Stapled peptides targeting LRRK2 dimers have been recently described, reducing dimer formation, kinase activity, and neuronal death. LRRK2 kinase inhibitors have already entered clinical trials for both LRRK2-PD as well as idiopathic PD (see Jennings D, Huntwork-Rodriguez S, Henry AG, Sasaki JC, Meisner R, Diaz D, Solanoy H, Wang X, Negrou E, Bondar VV, Ghosh R, Maloney MT, Propson NE, Zhu Y, Maciuca RD, Harris L, Kay A, LeWitt P, King TA, Kern D, Ellenbogen A, Goodman I, Siderowf A, Aldred J, Omidvar O, Masoud ST, Davis SS, Arguello A, Estrada AA, de Vicente J, Sweeney ZK, Astarita G, Borin MT, Wong BK, Wong H, Nguyen H, Scearce-Levie K, Ho C, Troyer MD (2022) Preclinical and clinical evaluation of the LRRK2 inhibitor DNL201 for Parkinson's disease. Sci Transl Med 14, eabj2658); and early safety trials have begun assessing the potential of LRRK2 antisense oligonucleotides as well. Considering that direct pharmacological inhibition of LRRK2 activity with ATP-competitive compounds can lead to pathological changes in the lung (see Baptista MAS, Merchant K, Barrett T, Bhargava S, Bryce DK, Ellis JM, Estrada AA, Fell MJ, Fiske BK, Fuji RN, Galatsis P, Henry AG, Hill S, Hirst W, Houle C, Kennedy ME, Liu X, Maddess ML, Markgraf C, Mei H, Meier WA, Needle E, Ploch S, Royer C, Rudolph K, Sharma AK, Stepan A, Steyn S, Trost C, Yin Z, Yu H, Wang X, Sherer TB (2020) LRRK2 inhibitors induce reversible changes in nonhuman primate lungs without measurable pulmonary deficits. Sci Transl Med 12**;** and Fuji RN, Flagella M, Baca M, Baptista MA, Brodbeck J, Chan BK, Fiske BK, Honigberg L, Jubb AM, Katavolos P, Lee DW, Lewin-Koh SC, Lin T, Liu X, Liu S, Lyssikatos JP, O'Mahony J, Reichelt M, Roose-Girma M, Sheng Z, Sherer T, Smith A, Solon M, Sweeney ZK, Tarrant J, Urkowitz A, Warming S, Yaylaoglu M, Zhang S, Zhu H, Estrada AA, Watts RJ (2015) Effect of selective LRRK2 kinase inhibition on nonhuman primate lung. Sci Transl Med 7, 273ra215) and a redistribution to microtubule-bound skein-like filaments (see Deniston CK, Salogiannis J, Mathea S, Snead DM, Lahiri I, Matyszewski M, Donosa O, Watanabe R, Bohning J, Shiau AK, Knapp S, Villa E, Reck-Peterson SL, Leschziner AE (2020) Structure of LRRK2 in Parkinson's disease and model for microtubule interaction. Nature 588, 344-349; and Kett LR, Boassa D, Ho CC, Rideout HJ, Hu J, Terada M, Ellisman M, Dauer WT (2012) LRRK2 Parkinson disease mutations enhance its microtubule association. Hum Mol Genet 21, 890-899), the development of alternative strategies to inhibit LRRK2 signaling may serve as complementary therapeutic approaches to disrupt LRRK2-induced neuronal loss.

### Materials and Methods

*Peptide Synthesis.* All synthesis solvents and reagents used were HPLC-grade and purchased from Fisher, Sigma-Aldrich, Novabiochem, or ChemPep. The peptides were synthesized using standard Fmoc solid phase peptide synthesis (SPPS). Synthesis was performed on a rink amide MBHA resin using standard Nα-Fmoc amino acids. To synthesize the peptides, the MBHA resin was first calibrated by agitation in 1mL of N-methylpyrrolidinone (NMP) for ten minutes. Peptides were then deprotected for twenty-five minutes with agitation. The deprotecting solution used contained 25% (v/v) piperidine and 75% (v/v) NMP. After each deprotection, the resin was washed three times for 30 seconds in NMP with agitation prior to coupling. Coupling was performed using ten equivalents of each Nα-Fmoc amino acid, 9.9 equivalents of 2-(6-chloro-1Hbenzotriazole-1-yl)-1,1,3,3-tetramethylaminium hexafluorophosphate (HCTU in NMP), and twenty equivalents of N,N-diisopropyl ethylamine (DIEA) as the catalyst for the reaction. Each amino acid coupling reaction was performed for forty-five minutes with agitation. To couple the olefinic amino acid, four equivalents of (S)-N-Fmoc-2-(4- pentenyl) alanine (Ss) was incorporated into the sequence using 3.9 equivalents HCTU and twenty equivalents DIEA. Olefinic amino acid coupling was performed for forty-five minutes with agitation. Following S₅ addition, each sequence was cyclized by ring closing metathesis (RCM). To perform the RCM, 1,2-dichloroethane (DCE) and 0.4 equivalents of 1^{st} generation Grubbs were added to the resin with agitation for two separate one-hour cycles. After cyclization, four equivalents of Fmoc-11-amino-3,6,9-trioxaundecanoic acid (PEG₃) was added to the resin with 3.9 equivalents of HCTU, and twenty equivalents of DIEA. To label each peptide, two equivalents of 5,6-carboxyfluorescein (FAM) in dimethylformamide (DMF), 1.8 equivalents of HCTU, followed by 4.6 equivalents of DIEA were added to each resin with overnight agitation. Following overnight labeling, peptides were cleaved from the resin using 95% (v/v) trifluoracetic acid (TFA), 2.5% (v/v) triisopropylsilane (TIS), and 2.5% (v/v) distilled water. To cleave the peptides from the resin, the solution was rotated for five hours at room temperature and precipitated via centrifugation at 4°C in methyl-tert-butyl ether. The supernatant was removed from each peptide and pellets were allowed to dry using continuous airflow overnight while protected from light.

*Peptide Characterization.* Following overnight drying, peptide pellets were diluted in 1 mL methanol and filtered using a 45um syringe filter. Peptides were then separated using an Agilent 1200 reverse-phase high-performance liquid chromatographer (RP-HPLC) on a Zorbax analytical SB-C18 column. For this RP-HPLC, the mobile phase linear gradient used was 10-100% water to acetonitrile and 0.1% TFA. The flow rate used was 0.5mL/min. To characterize the peptides, an Agilent 6120 Single Quadrupole electrospray-ionization mass spectrometer (ESI-MS) was used. Purification of each peptide was carried out over a semi-preparatory column with a flow rate of 4mL/min and with the same mobile phase as previously described. To confirm peptide purity, final ESI-MS assays were performed. Peptide molecular weights were used to characterize each product. The N-terminal intrinsic qualities were used to quantify the concentration of each peptide at 495nm. The sequences and molecular weights of each peptide are shown in Table 1.

**Table 1. The sequence and molecular weight of each peptide are listed below. Asterisks (*) symbolize S₅ residues.**

| **FAM-Labeled Peptide** | **Sequence** | **Molecular Mass (Predicted)** | **Molecular Weight (Observed)** |
|---|---|---|---|
| FARM 1 | 5(6)FAM-PEG3-K*ADL*QEVQQARDLQNRSK (SEQ ID NO. 2) | 2924.2 | 2924.4 |
| FARM2 | 5(6)FAM-PEG₃-KVADL*QEV*QARDLQNRSK (SEQ ID NO. 3) | 2895.2 | 2895.0 |
| FARM3 | 5(6)FAM-PEG3-KVADLVQE*QQA*DLQNRSK (SEQ ID NO. 4) | 2867.1 | 2866.8 |
| FARM4 | 5(6)FAM-PEG3-KVADLVQEVQQ*RDL*NRSK (SEQ ID NO. 5) | 2923.2 | 2922.6 |
| FAMR5 | 5(6)FAM-PEG3-KVADLVQEVQQA*DLQ*RSK (SEQ ID NO. 6) | 2852.2 | 2851.8 |

*Plasmids and cell culture.* Plasmids used for the assessment of the LRRK2-FADD PPI, as well as the over-expression of mutant LRRK2 in primary cortical neurons, were as described in Antoniou. Human LRRK2 cDNA was PCR-amplified and sub-cloned into pcDNA3.1(+) with an N-terminal Flag epitope tag. The G2019S and R1441C pathogenic mutations were introduced using site-directed mutagenesis (QuikChange; Agilent Technologies) as per the manufacturer's instructions, and fully sequenced to ensure no errors were introduced. N-terminal V5-tagged FADD expression constructs were used as previously described.

*Assessment of LRRK2-FADD interaction.* Co-immunoprecipitation was undertaken to determine the effects of the stapled peptides on the LRRK2-FADD PPI, as previously described. Briefly, 10cm plates of HEK293T cells transiently expressing Flag-WT or mutant LRRK2 and V5-FADD (at a 3:1 ratio), and treated with different concentrations of FARM peptides (as indicated in the Figure legends), were washed in PBS and lysed in co-IP buffer (20mM HEPES, pH7.4; 150 mM NaCl; 0.1% NP40; 2 mM EGTA; 2 mM MgCl₂; 10% glycerol, pH 7.2; protease and phosphatase cocktail [Agilent Technologies]) for 30 min on ice followed by 15 strokes with a Dounce glass homogenizer, and centrifugation at 13000 rpm for 15 min to remove cellular debris. Following pre-clearing with IgG-coupled beads, 40 µl of washed anti-Flag resin (Sigma-Aldrich) per mg of protein was added and the samples incubated under rotation overnight at 4°C. The following morning, the beads were centrifuged and washed a total of 5X with coIP buffer, before elution in 2X SDS sample buffer at 95°C for 10 min. The eluate (and input) was separated by SDS-PAGE and membranes probed with anti-Flag (LRRK2) and V5 (FADD).

*Assessment of LRRK2 kinase function.* To determine whether peptides targeting the LRRK2-FADD PPI could also alter kinase function, LRRK2 activity was assessed in a cellular setting, through the phosphorylation of an endogenous substrate (pT73-Rab10), as well as auto-phosphorylation (pS1292-LRRK2). HEK293T cells were transiently co-transfected (WT, G2019S-LRRK2 & Flag-Rab10) as above and treated with increasing concentrations of stapled peptide, or as a control, the selective kinase inhibitor MLi-2 (100nM, overnight). Parallel cultures were transfected with only WT or R1441C-LRRK2; and endogenous levels of phosphorylated RablO were assessed. Following 72hr of total expression, the cells were washed in cold PBS and collected. Cell lysis was performed using the following buffer: 50mM Tris HCl, pH 7.5; 1mM EGTA; 0.27 M sucrose; 1% Triton X-100; supplemented with protease and phosphatase inhibitor cocktails, and cell extracts stored at -80°C until use. The extracts were separated by SDS-PAGE and proteins transferred to nitrocellulose membranes, which were probed for total/pS1292-LRRK2, total/pT73-Rab10, or β-actin. For quantification, the band intensities for phosphorylated Rab 10 or LRRK2 were normalized to total expression of the respective protein.

*Assessment of Neuronal Death.* Embryonic day 16 (E16) pregnant C57BL mice were used in this example, with primary cortical neurons prepared as described previously. Briefly, cortices were removed and cut into small pieces followed by enzymatic digestion (trypsin 0.05% and 100 µg/ml DNAse) and mechanical dissociation. Cells were centrifuged and counted and plated on poly-D-lysine coated glass coverslips at a density of 150,000/cm² in BrainPhys neuronal culture medium (StemCell Technologies) supplemented with SM1 Neuronal Supplement (StemCell Technologies), L-glutamine (0.5 mM) and penicillin/streptavidin. After 3-4 DIV, neurons were transfected using Lipofectamine 2000 (ThermoScientific) as per the manufacturer's instructions. Neurons were transfected with Flag-tagged WT or mutant (G2019S) human LRRK2 (as described previously), or un-tagged LRRK2 expressed from the EGFP-pcms vector expressing EGFP under a separate promoter. The following day after transfection, we initiated the treatment of neurons with biotinylated (or fluorescent-tagged) stapled peptides (at 0.1 - 10 µM final concentration). We replenished the peptides in the neuronal medium after 24hr, at the indicated concentrations. After three days following transfection, and two days of treatment, the coverslips were washed in PBS and fixed in 3.7% paraformaldehyde for 20min at 4°C. The neurons were processed for immunofluorescence labeling with the following antibodies: GFP (chicken; Abeam), Flag (M2 mouse; Sigma-Aldrich), and DAPI nuclear stain. In the cases where biotinylated peptides were used, we labeled intracellular peptide using Alexa647-streptavidin (ThermoScientific). Mounted coverslips were imaged on a Leica TSP5 multi-photon confocal microscope, and the Z-stacks processed in FIJI/ImageJ, and Adobe Photoshop. For quantification of apoptotic neuronal profiles, we used the approach described by Antoniou and colleagues. Briefly, individual Flag-positive neurons were assessed by a research blinded to the experimental conditions. The corresponding DAPI signal was evaluated in Flag-positive neurons; and cells exhibiting condensed chromatin, fragmented into two or more "apoptotic bodies" were considered to be non-viable (i.e. apoptotic).

*Antibodies.* The following antibodies were used in this example: Flag (mouse clone Ml; Sigma-Aldrich), V5 (rabbit; Sigma-Aldrich), GFP (chicken; Abeam), active caspase-3 (rabbit; R&D Systems), phosphorylated (T73) Rab10 (rabbit clone MJF-R21; Abeam), total RablO (rabbit; Abeam), LRRK2 (mouse clone N241; NeuroMab), LRRK2 (rabbit clone UDD3; Abeam), pS935-LRRK2 (rabbit clone UDD2; Abeam), pS1292-LRRK2 (rabbit clone MJFR 19-7-8; Abeam).

The compositions and methods of the appended claims are not limited in scope by the specific compositions and methods described herein, which are intended as illustrations of a few aspects of the claims and any compositions and methods that are functionally equivalent are intended to fall within the scope of the claims. Various modifications of the compositions and methods in addition to those shown and described herein are intended to fall within the scope of the appended claims. Further, while only certain representative compositions and method steps disclosed herein are specifically described, other combinations of the compositions and method steps also are intended to fall within the scope of the appended claims, even if not specifically recited. Thus, a combination of steps, elements, components, or constituents may be explicitly mentioned herein; however, other combinations of steps, elements, components, and constituents are included, even though not explicitly stated.

## Claims

1. A synthetic polypeptide comprising an amino acid sequence having an α-helical shape that mimics a death domain of FADD,
wherein the polypeptide comprises at least one pair of non-natural amino acids inserted into the amino acid sequence,
wherein the pair of non-natural amino acids are cross-linked to stabilize the α-helical shape.

2. The synthetic polypeptide of claim 1, wherein the polypeptide comprises a variant of an amino acid sequence of SEQ ID NO. 1, wherein the variant comprises at least one pair of non-natural amino acids that are cross-linked to stabilize the α-helical shape.

3. The synthetic polypeptide of claim 1 or claim 2, wherein the at least one pair of non-natural amino acids are three, four, or seven amino acids apart.

4. The synthetic polypeptide of any one of claims 1 -3, wherein the at least one pair of non-natural amino acids independently comprise olefinic side chains that are cross-linked.

5. The synthetic polypeptide of any one of claims 1-4, wherein each of the non-natural amino acids are independently selected from: (S)-2-(2'-propenyl)alanine ("S3"); (S)-2-(4'-pentenyl)alanine ("S5"); (S)-2-(5'-hexenyl)alanine ("S6"); (S)-2-(7'-octenyl)alanine ("S8"); (R)-2-(2'-propenyl)alanine ("R3"); (R)-2-(4'-pentenyl)alanine ("R5"); (R)-2-(5'-hexenyl)alanine ("R6"); and (S)-2-(7'-octenyl)alanine ("S8").

6. The synthetic polypeptide of claim 5, wherein the at least one pair of non-natural amino acids comprises a pairing selected from the group consisting of an S5-S5 pairing, an S5-R8 pairing, an S8-R5 pairing, an R3-S6 pairing, an R6-S3 pairing, an R3-S5 pairing, and an R5-S3 pairing.

7. The synthetic polypeptide of claim 1, wherein the polypeptide is formed from a peptide comprising an amino acid sequence having at least 80% sequence similarity to a sequence selected from SEQ ID NO. 2, SEQ ID NO. 3, SEQ ID NO. 4, SEQ ID NO. 5, and SEQ ID NO. 6.

8. The synthetic polypeptide of claim 1, wherein the polypeptide is formed from a peptide comprising an amino acid sequence having at least 85%, at least 90%, at least 95%, or at least 99% sequence similarity to a sequence selected from SEQ ID NO. 2, SEQ ID NO. 3, SEQ ID NO. 4, SEQ ID NO. 5, and SEQ ID NO. 6.

9. The synthetic polypeptide of claim 1, wherein the polypeptide is formed from a peptide comprising an amino acid sequence selected from SEQ ID NO. 2, SEQ ID NO. 3, SEQ ID NO. 4, SEQ ID NO. 5, and SEQ ID NO. 6.

10. The synthetic polypeptide of claim 1, wherein the polypeptide is selected from: 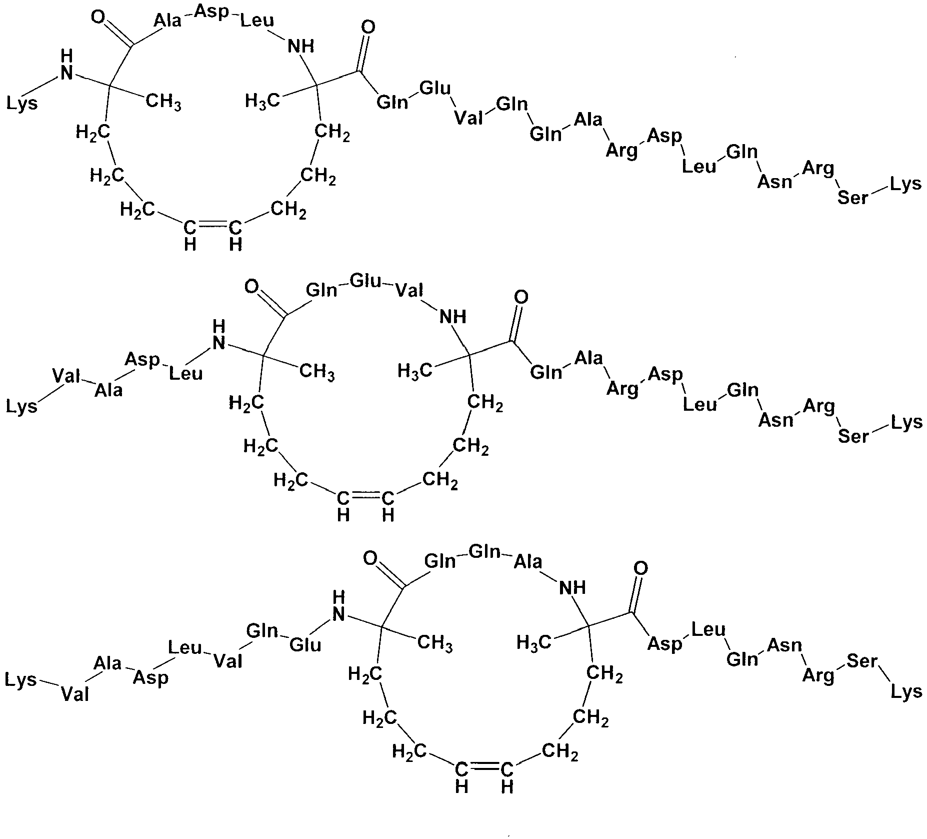 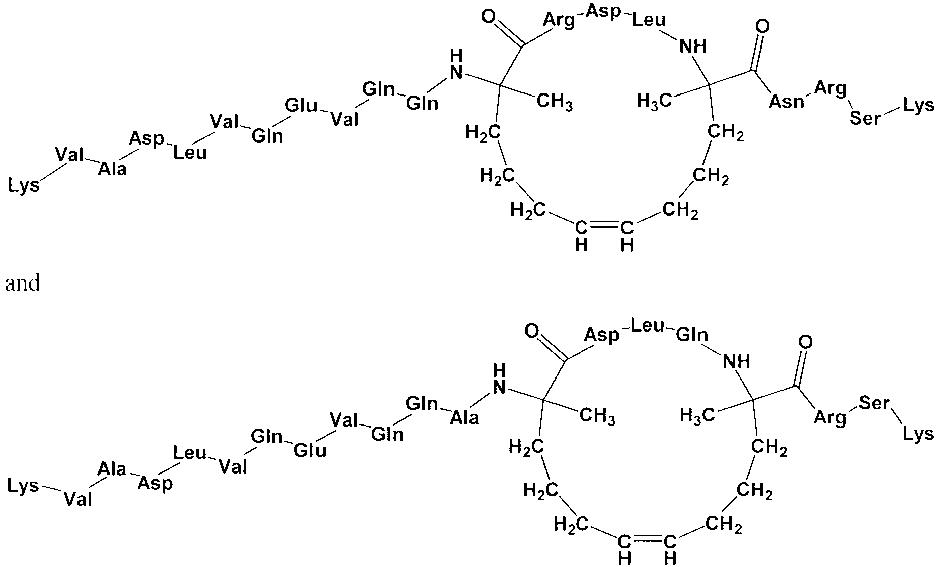 wherein
Ala is an alanine residue or a conservative substitution or a derivative thereof;
Arg is an arginine residue or a conservative substitution or a derivative thereof;
Asn is an asparagine residue or a conservative substitution or a derivative thereof;
Asp is an aspartate residue or a conservative substitution or a derivative thereof;
Gln is a glutamine residue or a conservative substitution or a derivative thereof;
Glu is a glutamate residue or a conservative substitution or a derivative thereof;
Leu is a leucine residue or a conservative substitution or a derivative thereof;
Lys is a lysine residue or a conservative substitution or a derivative thereof;
Ser is a serine residue or a conservative substitution or a derivative thereof; and
Val is a valine residue or a conservative substitution or a derivative thereof.

11. A pharmaceutical composition comprising the synthetic polypeptide of any one of claims 1-10 and a pharmaceutically acceptable carrier.

12. A cell comprising the synthetic polypeptide of any one of claims 1-10.

13. A synthetic polypeptide of any one of claims 1-10 for use in treating a neurological disease, disorder, or condition in a subject in need thereof.

14. The synthetic polypeptide for use of claim 13, wherein the neurological disease, disorder, or condition comprises Parkinson's disease, Huntington's disease, Alzheimer's disease, or amyotrophic lateral sclerosis (ALS).

15. A synthetic polypeptide of any one of claims 1-10 for use in treating a disease, disorder, or condition in a subject that is treated by inhibiting or decreasing protein-protein interactions between LRRK2 and FADD.
